# FASCICULE DE BREVET EUROPEEN

(11) **EP 0 768 307 B1**
(45) Date de publication et mention de la délivrance du brevet: **26.11.2003**
(21) Numéro de dépôt: 96402148.9
(22) Date de dépôt: 10.10.1996
(51) Int. Cl.: C07D 311/94, C07D 333/74, C07D 311/92, A61K 31/35, A61K 31/38

(54) **Amines hétérocycliques tertiaires, leur procédé de préparation et les compositions pharmaceutiques qui les contiennent**
Heterocyclisch tertiär Amine, Verfahren zu deren Herstellung und diese enthaltende pharmazeutischen Zusammenstellungen
Heterocyclic tertiary amines, process for their preparation and pharmaceutical compositions containing them

(30) Priorité: 13.10.1995 FR 9512044
(43) Date de publication de la demande: 16.04.1997
(73) Titulaire: LES LABORATOIRES SERVIER, 92200 Neuilly sur Seine (FR)
(72) Inventeur: Peglion, Jean-Louis, 78110 Le Vesinet (FR); Goument, Bertrand, 78220 Viroflay (FR); Harmange, Jean-Christophe, 78100 Saint Germain en Laye (FR); Vian, Joel, 92370 Chaville (FR); Dessinges, Aimée, 94310 Thiais (FR); Millan, Mark, 78230 Le Pecq (FR); Audinot, Valérie, 78300 Croissy (FR)

(56) Documents cités:
- EP-A- 0 481 243
- FR-A- 2 379 532

## Description

La présente invention a pour objet de nouvelles amines hétérocycliques tertiaires, leur procédé de préparation et les compositions pharmaceutiques qui les contiennent.

Elle concerne plus spécialement les amines hétérocycliques tertiaires de formule I : 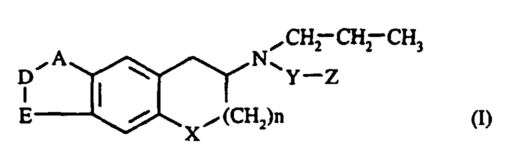 dans laquelle :
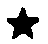 **-A-D-E-** représente 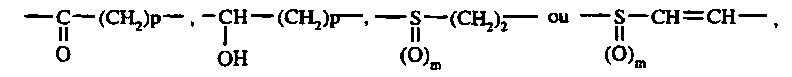 dans lesquels
   p représente 2 ou 3 et
   m représente zéro, 1 ou 2 ;
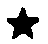 **X** représente :
   un groupe -CH₂- et de plus,
   lorsque -A-D-E- représente 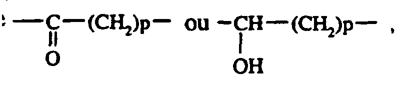 X peut aussi représenter un atome d'oxygène ;
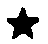 **n** représente :
   zéro ou 1 lorsque X représente le groupe CH₂, et
   uniquement 1 lorsque X représente un atome d'oxygène ;
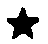 **Y** représente une liaison simple ou un groupe -CH₂-, et
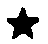 **Z** représente :
   un radical alkyle, alcényle ou alcynyle chacun contenant jusqu'à 10 atomes de carbone inclus, en chaîne droite ou ramifiée, et chacun éventuellement substitué par un ou plusieurs radicaux cycloalkyle ayant de 3 à 7 atomes de carbone inclus, ou
   un radical choisi parmi 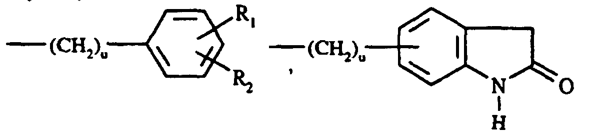 et 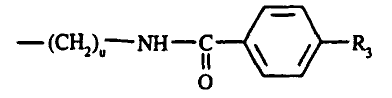 dans lesquels
   u représente un nombre entier de 1 à 6 inclus ;
   R₁ représente un atome d'hydrogène ou d'halogène, un radical hydroxy ou un radical alkyl ou alkoxy contenant chacun de 1 à 6 atomes de carbone inclus en chaîne droite ou ramifiée ;
   R₂ représente un atome d'halogène, un radical hydroxy, un radical alkyle ou alkoxy contenant chacun de 1 à 6 atomes de carbone inclus en chaîne droite ou ramifiée, un radical phényle, ou un groupement de formule NHCOO-C(CH₃)₃, NH₂, NH-COCH₃, NHCOCF₃ , NHSO₂CH₃ et 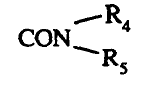 dans le quel R₄ et R₅, identiques
      ou différents, représentent chacun un atome d'hydrogène ou un radical (C₁-C₆)alkyle en chaine droite ou ramifiée ;
      et
   R₃ représente un atome d'halogène ou un radical hydroxy, (C₁-C₅)alkoxy en chaine droite ou ramifiée, trifluorométhyle, cyano, phényle, p-aminophényle ou p-acétylphényle;
sous forme racémique ou d'isomères optiques,
et leurs sels d'addition avec un acide pharmaceutiquement acceptable.

L'état antérieur de la technique est illustré notamment par les brevets EP 0 286 515 et 0 286 516 qui concernent, entre autres, des dérivés aminés du 5,6,7,8-tétrahydronaphto [2,3-b] furane se comportant comme des substances dopaminergiques et présentant une activité antidépressive, anti -agressive et psychostimulante.

Des recherches effectuées dans les services de la demanderesse ont montré qu'en modifiant d'une part les substituants de la fonction amine, et d'autre part la nature de l'enchainement A-D-E, on parvient à renforcer les propriétés dopaminergiques de ces produits tout en les rendant plus spécifiques et sélectives, ce qui permet aux dits produits de présenter moins d'effets secondaires.

Actuellement, les substances utilisées en thérapeutique pour le traitement des affections dans lesquelles est impliqué le système dopaminergique, ne présentent pas de sélectivité et se lient toutes très fortement au récepteur D₂, qu'il s'agisse des bloqueurs dopaminergiques (utilisés dans les troubles liés à l'hyperactivité de ce neurotransmetteur tels qu'ils se présentent, par exemple, dans la schizophrénie) ou d'activateurs dopaminergiques (utilisés dans les troubles liés à l'hypoactivité tels qu'ils se présentent dans la maladie de Parkinson par exemple).

Toutefois, ces bloqueurs ou activateurs dopaminergiques D₂ présentent de nombreux effets secondaires : dyskinésie tardive, hyperprolactinémie, aménorrhée pour les premiers, effets cardio-vasculaires et moteurs pour les derniers.

La découverte récente d'un nouveau récepteur à la dopamine, dénommé le récepteur D₃, dont la concentration est très importante au niveau du système limbique, mais très faible dans le noyau nigrostrié et dans les cellules lactotrophes, encourage la recherche de nouveaux médicaments agissant au niveau du système dopaminergique, mais en ayant pour cible préférentielle le récepteur D₃ et donc exempts des effets secondaires liés typiquement au récepteur D₂ comme mentionné précédemment.

Les modifications de structures des produits de l'état antérieur de la technique précédemment cité ont abouti aux composés objets de la présente invention qui se différencient des produits des brevets EP 0 286 515 et 0 286 516 à la fois par leur structure chimique et par leurs propriétés pharmacologiques et thérapeutiques.

En effet, des études réalisée in vitro (binding sur récepteurs clonés D₂ et D₃) avec les composés de la présente invention montrent que ceux-ci se comportent comme des ligands très affins au niveau des récepteurs dopaminergiques D₃ tout en ne présentant que peu d'affinité pour les récepteurs dopaminergiques D₂, ce qui n'est pas le cas des composés objets des brevets EP 0 286 515 et 0 286 516.

Cette sélectivité confère aux composés de la présente invention un intérêt tout particulier pour leur utilisation comme médicament agissant au niveau du système dopaminergique dans la maladie de Parkinson (J. Neur. Transm., 1993, 94, 11-19), les troubles de la mémoire (Nature, 1990, 347, 146-151), l'abus de drogue (Science, 1993, 260, 1814), la dépression et comme antipsychotique.

La présente invention a également pour objet le procédé de préparation des composés de formule I caractérisé en ce que :
∗ l'on fait réagir :
   . une amine secondaire de formule II : 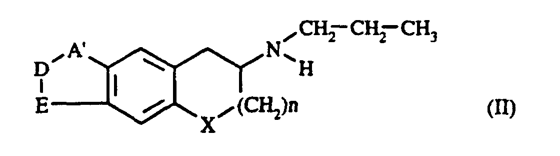 dans laquelle :
      X et n ont les significations précédemment définies et
      A'-D-E représente : (CH₂)₃, (CH₂)₄, -S-(CH₂)₂- ou -S-CH=CH-,
   . avec un composé de formule III :

      G-Y-Z (III)

      dans laquelle :
      Y et Z ont les significations précédemment définies et
      G représente un atome d'halogène, un radical mésyloxy ou un radical tosyloxy
∗ et l'on oxyde les composés de formule IV ainsi obtenus : 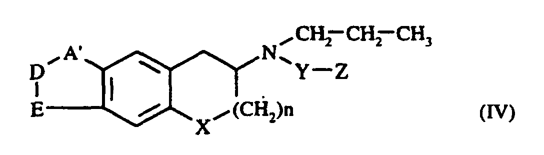 dans laquelle :
   -A'-D-E, X, n, Y et Z ont les significations précédemment définies au moyen :
      ∼ soit du réactif de Jones, ou de 2,3-dichloro-5,6-dicyano-1,4-benzoquinone dans acide acétique et eau, dans le cas où -A'-D-E- prend les valeurs (CH₂)₃ et (CH₂)₄,
      ∼ soit de l'eau oxygénée, dans le cas où -A'-D-E- prend les valeurs -S-(CH₂)₂- ou - S-CH=CH-,
∗ pour obtenir les composés de formule Ia : 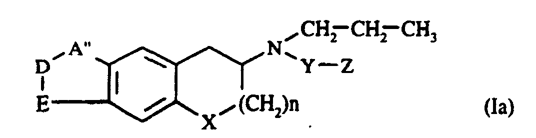 dans laquelle :
   X, n, Y et Z ont les significations précédemment définies et
   A"-D-E représente un enchaînement choisi parmi : 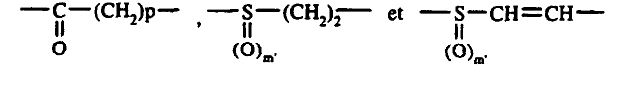 dans lesquels m' représente 1 ou 2, et p a la signification précédemment définie ;
∗ et dans le cas où A"-D-E représente 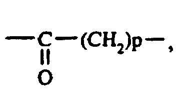 on réduit les composés de formule Ia correspondants [c'est-à-dire les composés répondant plus spécifiquement à la formule I'a : 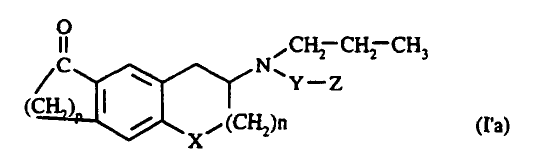 dans laquelle p, X, n, Y et Z ont les significations précédemment définies]
∗ pour obtenir les composés de formule Ib : 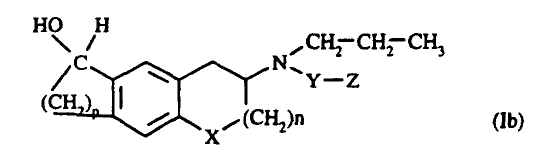 dans laquelle p, X, n, Y et Z ont les significations précédemment définies.

L'ensemble des composés de formule IV dans laquelle la signification de A'-D-E est uniquement -S-(CH₂)₂- et -S-CH=CH-, et des composés de formule Ia et Ib forme l'ensemble des composés de formule I.

La condensation des composés de formule II et III s'effectue avantageusement en présence de K₂CO₃/CH₃CN, K₂CO₃/H₂O, ou Na₂CO_{3/}CH₃COCH₃.

La réduction des composés de formule I'a est réalisée de façon particulièrement convenable en utilisant comme agent réducteur : LiAlH₄, NaBH₄ ou ZnBH₄.

De plus, les composés de formule I dans laquelle Y prend uniquement la valeur CH₂, c'est-à-dire les composés répondant plus spécifiquement à la formule I' : 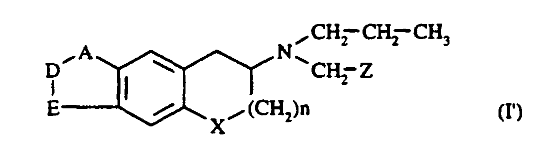 dans laquelle A-D-E, X, n et Z ont les mêmes significations que dans la formule I, ont également été préparés selon le procédé spécifique ci-après.

La présente invention a également pour objet le procédé de préparation des composés de formule I' : 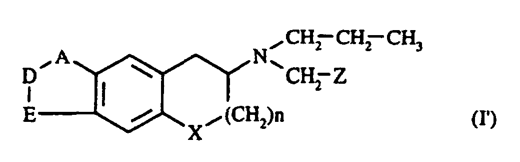 dans laquelle A-D-E, X, n et Z ont les significations précédemment définies,
- caractérisé en ce que l'on fait réagir l'amine secondaire de formule II précédemment définie, avec :
   . soit un composé de formule V : 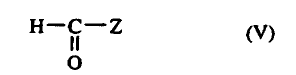
   dans laquelle Z a la signification précédemment définie,
   en opérant en milieu réducteur, par exemple en présence de triacétoxy borohydrure de sodium dans l'acide acétique;
   . soit un composé de formule VI : 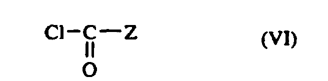 dans laquelle Z a la signification précédemment définie,
      et l'on réduit le composé obtenu de formule VII : 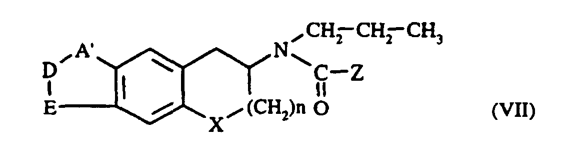 dans laquelle A'-D-E, X, n et Z ont les significations précédemment définies,
- afin d'obtenir, quelle que soit la voie réactionnelle utilisée, un composé de formule VIII : 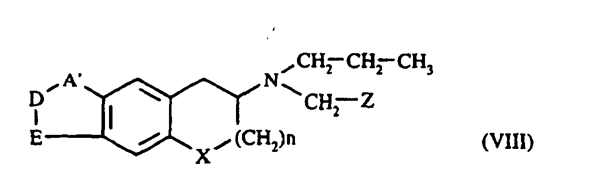 dans laquelle A'-D-E, X, n et Z ont les significations précédemment définies ;
- lequel composé de formule VIII est alors transformé (comme l'a été précédemment le composé de formule IV) par oxydation au moyen :
   . soit du réactif de Jones, ou de 2,3-dichloro-5,6-dicyano-1,4-benzoquinone dans acide acétique et eau, dans le cas où A'-D-E prend les valeurs (CH₂)₃ et (CH₂)₄,
   . soit de l'eau oxygénée, dans le cas où -A'-D-E- prend les valeurs -S-(CH₂)₂- ou -S-CH=CH,
- pour obtenir les composés de formule I"a : 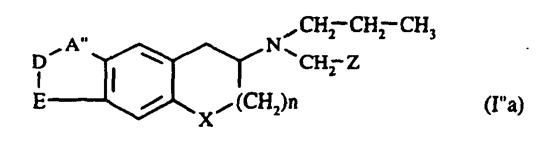 dans laquelle A"-D-E, X, n et Z ont les significations précédemment définies,
- et dans le cas où A"-D-E représente 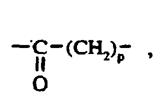 on réduit les composés de formule I"a correspondants [c'est-à-dire les composés correspondant plus spécifiquement à la formule I'''a : 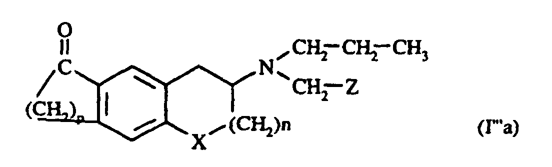 dans laquelle p, X, n et Z ont les significations précédemment définies]
   pour obtenir les composés de formule I'b : 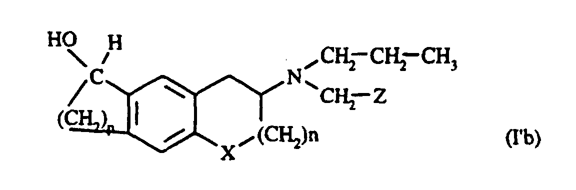 dans laquelle p, X, n et Z ont les significations précédemment définies.

L'ensemble des composés de formule VIII dans laquelle la signification de A'-D-E est uniquement -S-(CH₂)₂- et -S-CH=CH-, et des composés de formule I"a et I'b forme l'ensemble des composés de formule I'.

La condensation des composés de formule II et VI s'effectue avantageusement en présence de Na₂CO₃, H₂O et CH₃-COOC₂H₅. La réduction des composés de formule VII est réalisée de façon adéquate par le borane-sulfure de diméthyle [BH₃ . S(CH₃)₂] dans le tétrahydrofurane.

De plus, les composés de formule I dans laquelle -A-D-E- prend uniquement la valeur -S-CH=CH-, c'est à dire les composés répondant plus spécifiquement à la formule I" : 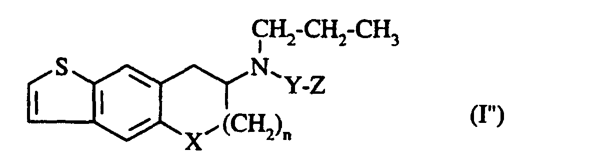 dans laquelle X, n, Y et Z ont les significations précédemment définies,
ont également été préparés selon le schéma opératoire suivant :

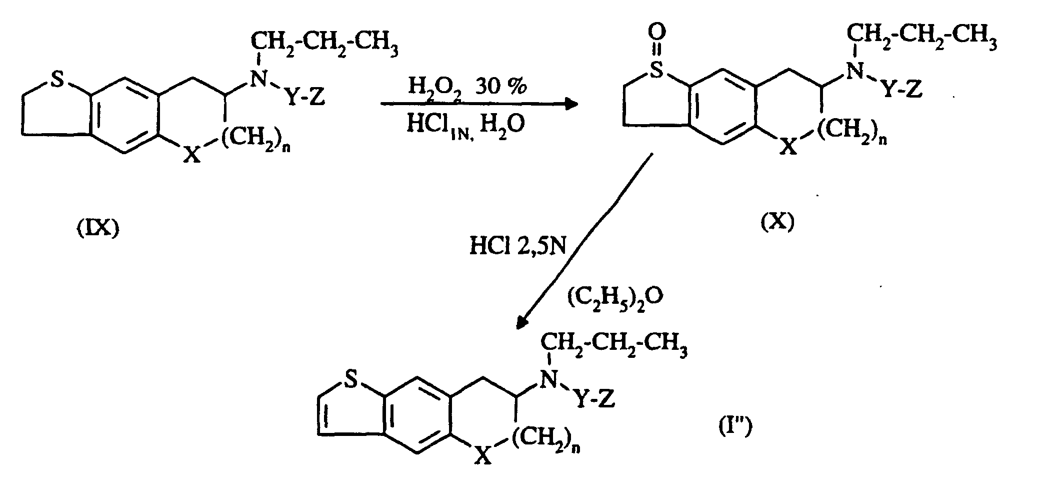

D'autre part, les composés de formule I dans laquelle -A-D-E- prend uniquement la valeur 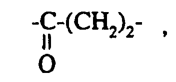 c'est à dire les composés répondant plus spécifiquement à la formule I''' : 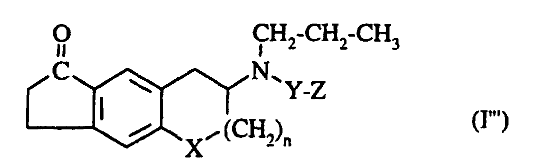 dans laquelle X, n, Y et Z ont les significations précédemment définies,
ont également été préparés avantageusement selon le procédé ci-après qui fait également partie de la présente invention.

La présente invention a donc aussi pour objet le procédé de préparation des composés de formule I''' : 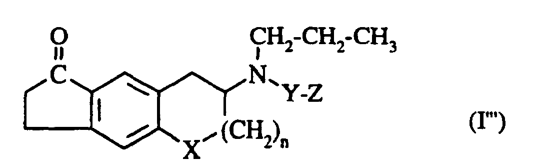 dans laquelle X, n, Y et Z ont les significations précédemment définies,
caractérisé en ce que :
l'on traite les composés de formule XI : 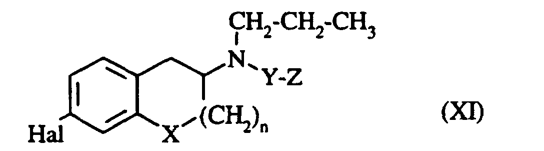 dans laquelle X, n, Y et Z ont les significations précédemment définies, et Hal représente un atome d'halogène, et plus particulièrement un atome de brome et d'iode,
au moyen d'acrylate de méthyle dans le diméthylformamide en présence d'acétate de palladium, de tri-o-tolylphosphine et de triéthylamine,
pour donner les composés de formule XII : 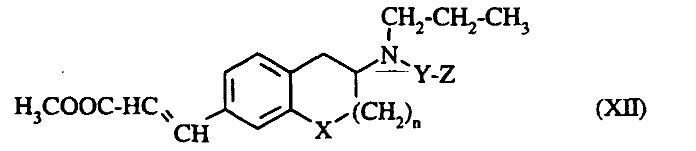 dans laquelle X, n, Y et Z ont les significations précédemment définies,
que l'on réduit par l'hydrogène en présence de platine sur charbon pour conduire aux composés de formule XIII : 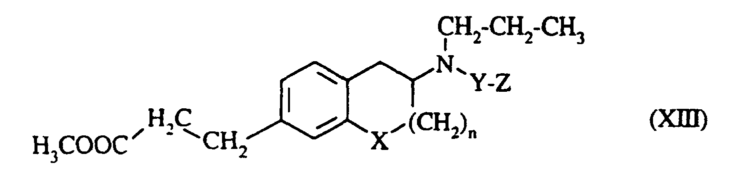 dans laquelle X, n, Y et Z ont les significations précédemment définies,
que l'on traite par la soude pour donner les composés de formule XIV : 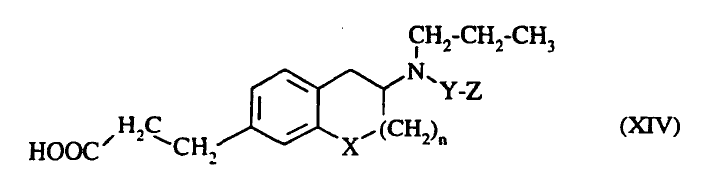 dans laquelle X, n, Y et Z ont les significations précédemment définies,
lesquels sont cyclisés par action de l'anhydride phosphorique pour obtenir les composés de formule I''' précédemment définie.

Les formes optiquement actives des composés de formule I ont été obtenues soit à partir des formes optiquement actives des matières premières de formule II, soit par dédoublement des formes racémiques des composés de formule I, selon des méthodes connues de la littérature.

Les sels des composés de formule I avec des acides pharmaceutiquement acceptables ont été obtenus selon des méthodes classiques comme indiqué dans les exemples ci-après.

Les matières premières de formule II sont obtenues selon les méthodes ci-après décrites pour la préparation des matières premières des produits exemplifiés.

La présente invention a également pour objet les compositions pharmaceutiques contenant comme principe actif un composé de formule I ou un de ses sels physiologiquement tolérable mélangé ou associé à un ou plusieurs excipients pharmaceutiques appropriés.

Les compositions pharmaceutiques ainsi obtenues se présentent généralement sous forme dosée renfermant de 0,5 à 25 mg de principe actif. Elles peuvent, par exemple, revêtir la forme de comprimés, dragées, gélules, suppositoires, solutions injectables ou buvables et être administrées par la voie orale, rectale ou parentérale.

La posologie peut varier selon l'âge et le poids du patient, la voie d'administration, la nature de la maladie et les traitements associés et s'échelonne de 0,5 à 25 mg de principe actif, 1 à 3 fois par jour.

Les exemples suivants, donnés à titre non-limitatif, illustrent la présente invention.

Les points de fusion ont été déterminés soit à la platine chauffante de Kofler (K), soit à la platine chauffante sous microscope (MK).

Les spectres de résonnance magnétique nucléaire du proton (RMN) ont été réalisés à 200 MHz.

### Synthèse des matières premières

Les matières premières utilisées dans les exemples suivants ont été préparées comme suit :

### Préparation 1

### Chlorhydrate de (3RS)-3-(N-propylamino)cyclopenta[g]3,4-dihydro-2H-benzopyrane

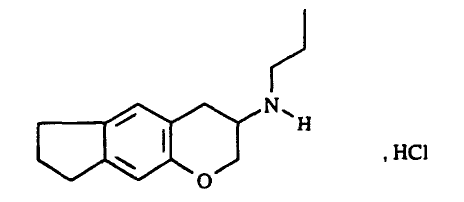

### Stade A : (3RS)-3- nitro cyclopenta[g]2H-benzopyrane

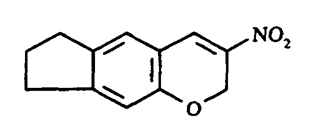

A température ambiante, on mélange 34,7 g (214 mmole) de 6-hydroxyindan 5-carboxaldéhyde [décrit dans J.A.C.S 1955, 77, 2466-75], 36,7 g (342,3 mmole) de nitroéthanol technique (à 85 %), 17,7 g (107 mmole) de chlorhydrate de di-n-butylamine et 430 ml d'acétate d'isoamyle. On porte à reflux 24 h, puis laisse refroidir. On filtre le solide qui s'est formé, le rince à l'acétate d'éthyle, joint les filtrats et évapore à sec. Le résidu est repris par 500 ml de dichlorométhane et lavé par 200 ml d'eau, 200 ml de soude N (2 fois) et 200 ml d'eau, puis séché sur sulfate de magnésium. Après évaporation, puis flash-chromatographie sur 1,5 Kg de silice (éluant : dichlorométhane), on obtient 11,3 g du dérivé souhaité sous forme d'un solide orange. PF (K) : 156 °C. Rendement : 24 %.

### Stade B : (3RS)-3-nitro cyclopenta[g]3,4-dihydro-2H-benzopyrane

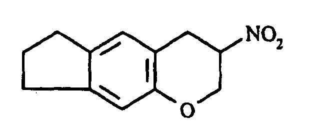

A 11,3 g (51,6 mmole) du composé précédent solubilisé dans un mélange de 330 ml de chloroforme et 110 ml d'isopropanol, sous agitation énergique, on ajoute d'un trait 24,5 g de silice (Merck 230-400 mesh), puis par fractions en 15 mn, 4,7 g (124,2 mmole) de borohydrure de sodium. On agite 30 mn à température ambiante, puis bloque la réaction par addition de 8,5 ml d'acide acétique. On agite encore 15 mn, puis filtre sur verre fritté et rince abondamment au dichlorométhane. Les filtrats joints sont évaporés pour obtenir 11,8 g du dérivé nitro attendu sous forme d'un solide jaune. PF (K) : 141 °C. Rendement quantitatif.

### Stade C : (3RS)-3-amino cyclopenta[g]3,4-dihydro-2H-benzopyrane

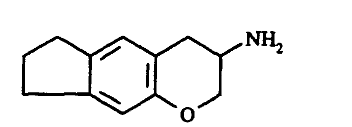

A 11,8 g (53,8 mmole) du composé précédent dissout dans 270 ml d'acide acétique, on ajoute 35,2 g (538 mmole) de zinc en poudre. On porte à 100 °C pendant 45 mn, puis refroidit, filtre le zinc et le rince abondamment au dichlorométhane. Après évaporation des filtrats, le résidu est repris par 250 ml de dichlorométhane. On extrait par 60 ml d'acide chlorhydrique N (3 fois), joint les phases aqueuses acides et les basifie à froid par de la soude concentrée, puis extrait par 100 ml de dichlorométhane (2 fois). Les phases organiques jointes sont séchées sur sulfate de magnésium, puis concentrées pour donner 3,45 g de l'amine attendue sous forme d'un solide beige. PF (K) : 61-63 °C. Rendement :34 %.

Pendant le passage acide-base, un insoluble blanc s'est formé. Il a été filtré et séché, puis caractérisé comme étant le chlorhydrate de l'amine attendue.

### Stade D : (3RS)-3-propionamido cyclopenta[g]3,4-dihydro-2H-benzopyrane

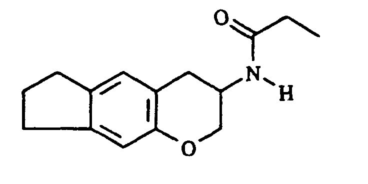

A 8,5 g (45 mmole) de l'amine obtenue au stade précédent dans 215 ml d'acétate d'éthyle, à température ambiante, on ajoute 215 ml d'une solution aqueuse à 10 % de carbonate de sodium puis 5,85 ml (67 mmole) de chlorure de propionyle goutte à goutte ; puis on maintient la nuit sous agitation à température ambiante. Le solide blanc formé est filtré sur verre fritté, rincé à l'eau et séché sous vide pour donner 7,16 g de l'amide attendu. PF (K) : 162 °C. Rendement : 65 %.

### Stade E : produit titre

On solubilise dans 80 ml de tétrahydrofurane, 7 g (28,6 mmole) du composé obtenu au stade D. On coule goutte à goutte 13,56 ml de borane-diméthylsulfure ( 143 mmole) et porte 20 h à reflux. On refroidit puis ajoute 30 ml de CH₃OH et porte de nouveau 3 h à reflux. On évapore les solvants et reprend le résidu par 150 ml d'HCl 1N et 150 ml d'éther. On filtre le précipité et le lave à l'éther. On obtient 6,05 g de produit attendu. PF (K) :> 260 °C. Rendement : 80 %.

### Préparation 2

### Chlorhydrate de (3RS)-3-(N-propylamino)cyclohexa[g]3,4-dihydro-2H-benzopyrane

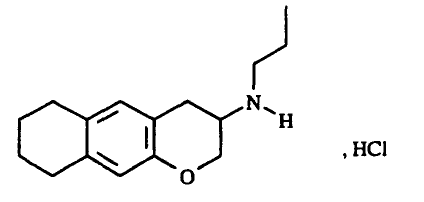

En procédant comme décrit pour la préparation 1, stades A à E mais en utilisant au stade A le 5,6,7,8-tétrahydro 3-hydroxynaphtalèn 2-carboxaldéhyde (cf. J.A.C.S, 1958, 80, 3294-3300) à la place du 6-hydroxyindan 5-carboxaldéhyde, on obtient le chlorhydrate attendu. PF(K) > 260 °C.

### Préparation 3

### (3RS)-3-(N-propylamino) 6-oxocyclopenta[g]3,4-dihydro-2H-benzopyrane

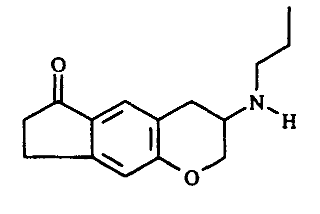

1g (3,7 mmole) du composé titre de la préparation 1 est mis en suspension dans 15 ml d'acétone à 0 °C. On ajoute goutte à goutte 3,7 ml de réactif de Jones en maintenant à une température inférieure à 4 °C, puis on agite 4 h à température ambiante. On refroidit à nouveau à 0 °C, ajoute 2 ml d'isopropanol et laisse la nuit sous agitation à température ambiante. Les sels sont filtrés et la phase organique est évaporée. Le résidu est repris par 25 ml d'HCl 0,5N et lavé deux fois par 30 ml d'éther. La phase aqueuse acide est basifiée par NaOH concentrée puis extraite à l'éther. Après lavage et séchage de la phase organique, on obtient 500 mg du composé désiré. PF (K) : 84 °C. Rendement : 78 %.

RMN (CDCl₃/TMS) : 7,5 (s,1H) ; 6,8 (s,1H) ; 4,25 (dd,1H) ; 4,0 (s,1H) ; 3,15 (m,1H) ; 3,0 (d,2H) ; 2,8 à 2,6 (m,6H) ; 1.7 (massif échangeable par D₂O) ; 1,5 (m,2H) ; 0,95 (t,3H).

### Préparation 4

### Chlorhydrate de (7RS)-7-(N-propylamino)-2,3,5,6,7,8-hexahydronaphto[2,3-b]thiophène

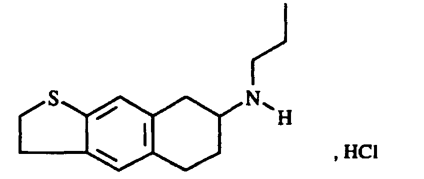

### Stade 1 : 2,3,5,6,7,8-hexahydro 7-hydroxyimino 8-oxonaphto[2,3-b]thiophène

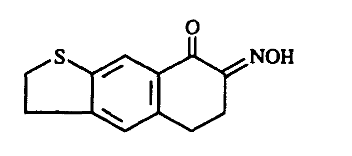

Dans un ballon tricol de 4 litres muni d'une agitation mécanique et d'une entrée d'azote, 53,2 g (474 mmole) de tert-butylate de potassium sont mis en suspension dans 724 ml d'éther éthylique sec. A cette solution sont successivement additionnés à température ambiante 95 ml d'alcool tert-butylique et 95 ml d'alcool éthylique. La solution ainsi obtenue est portée à reflux 1 h puis refroidie à 3 °C. A cette température le nitrite d'isoamyle 95 ml et le 2,3,5,6,7,8-hexahydro-8-oxonaphto[2,3-b]thiophène [préparé selon W. Carruthers et coll. ; J Chem. Soc., 1962, p 704-708] (77,38 g, 379 mmole) en solution dans le mélange ether éthylique/tétrahydrofurane (1500 ml/200 ml) sont successivement additionnés. Après 0,5 h d'agitation à 3 - 5 °C, le mélange réactionnel est rapidement filtré sous azote. Le précipité est rincé une fois par de l'éther éthylique anhydre et rapidement additionné par portion à une solution aqueuse d'HCl 1N refroidie à l'aide d'un bain eau-glace tout en agitant vigoureusement. Le précipité est filtré, rincé à l'eau et séché sous vide pour donner une poudre de couleur sombre : 59,3 g. Rendement: 67 %.

### Stade 2 :2,3,5,6,7,8-hexahydro 7-(N-propionamido) 8-oxonaphto[2,3-b]thiophène

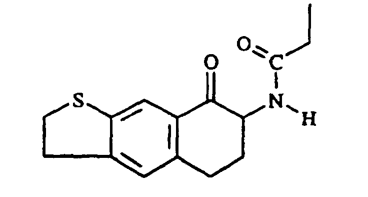

Dans un ballon tricol de 3 litres muni d'une agitation mécanique, 59 g (82,9 mmole) de l'oxime précédente sont mis en solution dans 800 ml d'un mélange 1/1 acide propionique/anhydride propionique. Après 20 mn d'agitation à température ambiante, 67 g de zinc sont ajoutés par portion (1 h). 30 mn après la fin de l'addition, le mélange réactionnel est dilué par 250 ml de CH₂Cl₂ puis filtré. Le précipité est lavé par 250 ml de CH₂Cl₂ puis filtré et le filtrat est concentré sous vide pour donner 137 g d'un solide rouge sombre. La purificaton de ce dernier par chromatographie sur silice (CH₂Cl₂/acétate d'éthyle : 90/10) permet d'obtenir 35 g du produit attendu. PF(K) : 158-160 °C. Rendement : 50 %.

### Stade 3 : 2,3,5,6,7,8-hexahydro 7-(N-propionamido) 8-hydroxynaphto[2,3-b]thiophène

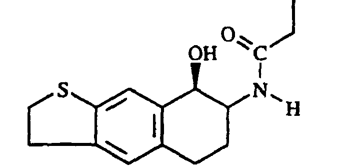

Dans un ballon tricol de 4 litres muni d'une agitation mécanique et d'une entrée d'azote, 3,8 g (99,5 mmole) d'AlLiH₄ sont mis en suspension dans 850 ml de tétrahydrofurane sec. à -78 °C, 22,8 g (253 mmole) du produit obtenu précédemment en solution dans 1060 ml de tétrahydrofurane sec sont lentement additionnés en maintenant la température du mélange réactionnel en dessous de -70 °C. Après 2 h d'agitation à -78 °C, 2,5 ml d'une solution aqueuse saturée de NH₄Cl sont lentement additionnés et le mélange réactionnel est ramené à température ambiante. Le précipité est filtré et copieusement rincé par du tétrahydrofurane. Le filtrat est concentré sous vide. Le résidu (meringue de couleur sombre) est purifié par chromatographie sur silice (éluant : CH₂Cl₂/acétate d'éthyle : 90/10) pour donner 15 g du produit attendu. Rendement : 65 %.

### Stade 4 : 2,3,5,6,7,8-hexahydro 7-(N-propionamido)naphto[2,3-b]thiophène

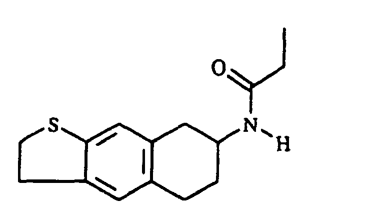

Dans un ballon tricol de 500 ml muni d'une agitation mécanique, d'un réfrigérant et d'une entrée d'azote, 3 g (10,8 mmole) du produit préparé précédemment sont mis en suspension dans 72 ml de CCl₄, 72 ml d'hydrure de triéthylsilane puis 35 ml d'acide trifluoroacétique sont additionnés et le mélange réactionnel est porté à reflux. Après 18 h d'agitation à reflux, le mélange réactionnel est concentré sous vide. Le résidu est repris par 100 ml d'acétonitrile et 100 ml d'hexane. La phase hexane est extraite trois fois par 50 ml d'acétonitrile. Les phases acétonitrile sont rassemblées, lavées une fois par 50 ml d'hexane et concentrées sous vide. Le résidu est purifié par chromatographie sur silice (éluant : CH₂Cl₂/acétate d'éthyle : 90/10) pour donner 2,19 g du produit attendu. PF(K) : 150-152 °C. Rendement : 83 %.

### Stade 5 : produit titre

Dans un ballon bicol de 250 ml muni d'une agitation magnétique, d'un réfrigérant et d'une entrée d'azote, 2,16 g (8,27 mmole) de l'amide précédente sont mis en solution dans 95 ml de tétrahydrofurane sec. 8,3 g (8,3 mmole) de borane diméthylsulfure sont lentement additionnés à température ambiante. Après 18 h d'agitation à reflux, la réaction est stoppée par addition lente de méthanol en refroidissant le mélange réactionnel avec un bain eau-glace. Après 0,5 h d'agitation à température ambiante, le mélange réactionnel est concentré sous vide. Le résidu est repris par 150 ml de méthanol et chauffé à reflux 2 h en présence de 0,5 ml de HCl aqueux à 37 %. Cette solution méthanolique est concentrée sous vide pour donner 2,48 g du produit attendu. PF (K) : 240-250 °C avec décomposition. Rendement quantitatif.

### Préparation 5

### (2 RS)-2-(N,N-dipropylamino)cyclopenta[g]1,2,3,4-tétrahydronaphtalène

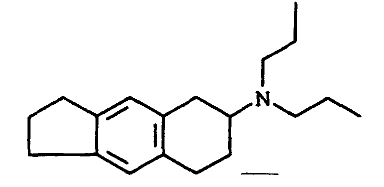

### Stade 1 : cyclopenta[f]indan-1-one

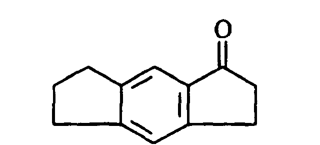

Un mélange de 31 g (0,163 mole) d'acide 3-(indan-5-yl)propionique et de 154,9 g d'acide polyphosphorique est chauffé pendant 1 h à 80 °C. Après refroidissement, on verse sur de la glace et laisse agiter une nuit. On extrait au dichlorométhane, lave, sèche sur MgSO₄ et évapore. Après purification sur silice par flash chromatographie avec un mélange CH₂Cl₂/cyclohexane 30/70 puis 70/30, on obtient 6,5 g de produit attendu. Rendement : 23%.

### Stade 2 : 1-aminométhyl cyclopenta[f]indan-1-ol

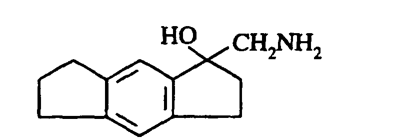

On introduit dans un autoclave 6 g (0,034 mole) du produit obtenu au stade 1, 5,19 g (0,052 mole) de cyanure de triméthylsilyle, 1,39 g d'iodure de zinc et 32,7 ml de 1,2-diméthoxyéthane et on porte le tout à 60 °C pendant 4 jours. Après refroidissement, la solution obtenue est ajoutée goutte à goutte à une suspension de 2,55 g d'hydrure de lithium et d'aluminium dans 70,4 ml de glyme anhydre. A la fin de l'addition, le milieu réactionnel est porté à 60 °C. Le chauffage est prolongé pendant une nuit. Puis le mélange réactionnel est hydrolysé par 1,78 ml d'eau, 1,428 ml de soude à 20 % et enfin 6,5 ml d'eau. Après filtration, le solvant est évaporé et le résidu est repris à l'éther. Une extraction acido-basique donne 4,04 g de produit attendu. Rendement : 58,5 %.

### Stade 3 : 2-oxo cyclopenta[g] 1,2,3,4-tétrahydronaphtalène

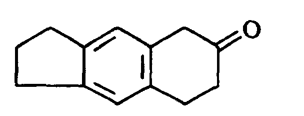

2g (9,8 mmole) de produit obtenu au stade précédent est dissout dans 20 ml d'une solution d'acide acétique à 25 % dans l'eau. Après refroidissement à 0 °C, on coule goutte à goutte une solution de 1,66 g de nitrite de sodium dans 7 ml d'eau. Dès l'addition terminée, on laisse revenir à température ambiante et agite pendant 3 h. Après extraction du milieu réactionnel par le dichlorométhane, la phase organique est lavée à la soude 0,1 N puis à l'eau. Après séchage et évaporation, on obtient 1,4 g de produit attendu. Rendement : 76,7 %.

### Stade 4 : produit titre

Dans un tricol équipé d'un Dean-Stark, on place 1,4 g (7,5 mmole) du produit obtenu au stade précédent, 50 ml de benzène et une pointe de spatule d'acide para-toluènesulfonique. Le milieu réactionnel est porté à reflux et on y ajoute 3,53 g (34,6 mmole) de dipropylamine dissous dans 5 ml de benzène. Le reflux est poursuivi pendant 48 h. Après évaporation du benzène, on obtient 2,3 g de résidu qui est immédiatement repris par 23 ml d'éthanol et 10 ml d'acide acétique. Après addition de 0,23 g de palladium sur charbon à 10 %, on hydrogène le milieu réactionnel à température ambiante et sous pression atmosphérique. On filtre, on évapore et après traitement acide-base, on obtient 0,49 g de produit attendu. Rendement : 21 %.

### Example 1

***(3RS)-3-(N,N-dipropylamino) 6-oxocyclopenta[g]3,4-dihydro-2H-benzopyrane*** 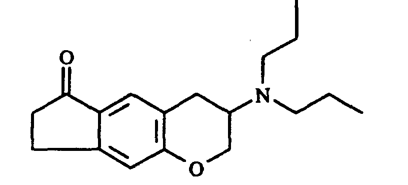 ***et son chlorhydrate***

### Stade 1 : (3RS)-3-(N,N-dipropylamino)cyclopenta[g]3,4-dihydro-2H-benzopyrane

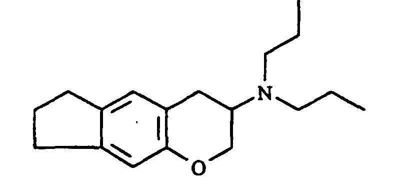

A 0,9 g (3,89 mmole) de la base libre correspondant au chlorhydrate obtenu dans la préparation 1, en solution dans 20 ml d'acétonitrile, on ajoute 1,39 g de K₂CO₃. On coule ensuite 0,5 ml (5,05 mmole) de 1-iodopropane, puis porte 24 h à reflux. On filtre et évapore le solvant. On reprend par de la soude concentrée et extrait au chlorure de méthylène pour obtenir 0,7 g de produit attendu sous forme d'huile. Rendement : 64 %.

### Stade 2 : Produit titre

A 5,3 g (19,4 mmole) de l'amine précédente dans 70 ml d'acétone, à 0 °C, on ajoute goutte à goutte 19,4 ml de réactif de Jones, puis agite pendant 20 h à température ambiante. On ramène à 0°C, ajoute goutte à goutte 10 ml d'isopropanol, puis agite 6 h à température ambiante. On filtre les sels de chrome sur verre fritté, rince à l'acétone et concentre les filtrats. Le résidu est repris par 130 ml d'acide chlorhydrique 0,5 N, on lave par 140 ml d'éther (2 fois), puis basifie à froid par de la soude concentrée. On extrait à l'éther, lave les phases organiques jointes à l'eau, les sèche sur sulfate de magnésium et concentre pour obtenir 3,44 g de l'amine attendue. Rendement 62 %.

0,23 g de cette amine est solubilisé dans un mélange ether/tétrahydrofurane. On ajoute 0,25 ml (1,1 éq.) d'éther chlorhydrique 2,5N, filtre le solide formé, le rince à l'éther et le sèche sous vide pour obtenir 0,23 g du chlorhydrate. PF (MK) : 161-164 °C. Rendement : 89 %.

RMN (DMSO-d6/TMS) : 10,95 (massif échangeable par D₂O) ; 7,5 (s,1H) ; 7,0 (s,1H) ; 4,65 (m,1H) ; 4,5 (m,1H) ; 3,9 (m,1H) 3,4 (m,2H) ; 2,9 à 3,2 (m,6H) ; 2,6 (t,2H) ; 1,75 (m,4H) ; 0,9 (t,6H).

### Exemple 2

***(3RS)-3-(N-propyl N-isopentylamino) 6-oxocyclopenta[g]3,4-dihydro-2H-benzopyrane*** 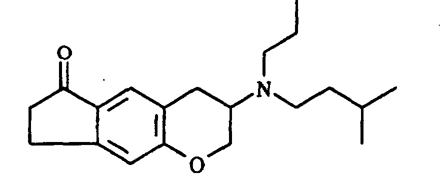 ***et son chlorhydrate***

En procédant comme décrit à l'exemple 1 mais en utilisant au stade 1 le 1-iodo 4-méthyl butane à la place du 1-iodopropane, on obtient le chlorhydrate du produit titre. PF(MK) : 88-91 °C.

RMN (DMSO-d6/TMS) : 11,05 (massif échangeable par D₂O) ; 7,5 (s,1H) 7,0 (s,1H) ; 4,7 (m,1H) ; 4,5 (m,1H) ; 3,9 (m,1H) ; 3,0 à 3,5 (m,8H) ; 2,65 (t,2H) ; 1,5 à 1,9 (m,5H) ; 0,9 (d+t,9H).

### Exemple 3

***(3RS)-3-(N,N-dipropylamino) 6-oxocyclohexa [g] 3,4-dihydro-2H-benzopyrane*** 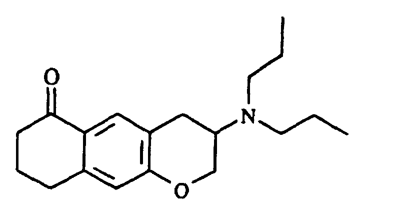 ***et son chlorhydrate*.**

En procédant comme décrit à l'exemple 1 mais en utilisant le produit obtenu à la préparation 2, on obtient le chlorhydrate du produit titre. PF(MK) : 181-184 °C.

RMN(DMSO-d6/TMS) : 10,85 (massif échangeable par D₂O) ; 7,75 (s,1H) ; 6,8 (s,1H) ; 4,65 (dd,1H) ; 4,4 (dd,1H) ; 3,85 (m,1H) ; 3,0 à 3,4 (m,6H) ; 2,9 (t,2H) ; 2,5 (t,2H) ; 2,0 (m,2H) ; 1,75 (m,4H) ; 0,9 (t,6H).

### Exemple 4

***(7RS)-7-(N,N-dipropylamino) 2,3,5,6,7,8-hexahydronaphto[2,3-b]thiophène*** 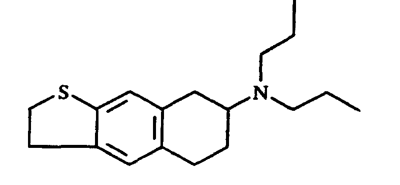 ***et son chlorhydrate***

### Stade 1 : (7RS)-7-(N-propyl N-propionamido) 2,3,5,6,7,8-hexahydronaptho[2,3-b]thiophène

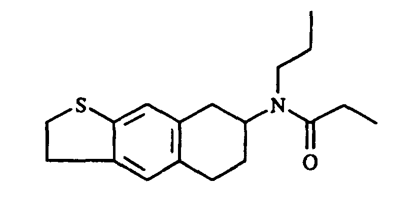

Dans un ballon tricol de 250 ml muni d'une agitation mécanique, 2,4 g (8,46 mmole) du chlorhydrate d'amine obtenu à la préparation 4 sont mis en suspension dans 63 ml d'acétate d'éthyle. 63 ml d'une solution aqueuse à 5 % de Na₂CO₃ sont additionnés à température ambiante et le mélange biphasique est vigoureusement agité jusqu'à dissolution totale. A cette solution biphasique, 1,1 ml (12,7 mmole) de chlorure de propionyle sont ajoutés à température ambiante. Après 5 h d'agitation à température ambiante, les phases organique et aqueuse sont séparées et la phase aqueuse est extraite trois fois par de l'acétate d'éthyle. Les phases organiques sont rassemblées, séchées sur sulfate de magnésium et concentrées pour donner 2,51 g de l'amide attendue. Rendement : 97 %.

### Stade 2 : produit titre

Dans un ballon bicol de 500 ml muni d'une agitation magnétique, d'un réfrigérant et d'une entrée d'azote, 2,51 g (8,28 mmole) de l'amide précédente sont mis en solution dans 95 ml de tétrahydrofurane sec. 8,28 ml (83 mmole) de borane-sulfure de diméthyle sont lentement additionnés à température ambiante. Après 24 h d'agitation à reflux, la réaction est stoppée par addition lente de méthanol en refroidissant le mélange réactionnel avec un bain eau-glace. Après 0,5 h d'agitation à température ambiante, le mélange réactionnel est concentré sous vide. Le résidu est repris par 100 ml de méthanol et chauffé à reflux 2 h en présence de 0,5 ml de HCl aqueux à 37 %.
La solution méthanolique est concentrée sous vide et le résidu est partagé entre 100 ml d'éther éthylique et 100 ml de NaOH aqueux 1N. La phase aqueuse est extraite quatre fois par 60 ml d'éther éthylique. Les phases organiques sont rassemblées, lavées par de la saumure, séchées sur sulfate de magnésium et concentrées pour donner 0,8 g d'amine. L'addition d'éther chlorhydrique à une solution de cette dernière dans 10 ml d'éther éthylique permet d'obtenir le chlorhydrate du produit titre. PF(MK) : 180-183 °C. Rendement : 30 %.

RMN ¹H (DMSOd6/TMS),δ : 10,6 (NH⁺), 7,05 (2s,2H) ; 3,6 (m,1H) ; 3,3-2,7 (m,10H) ; 2,3 (m,1H) ; 1,8 (m,5H) ; 0,9 (t,6H).

### Exemple 5

***(7RS)-7-(N-isopentyl N-propylamino)-2,3,5,6,7,8-hexahydronaphto[2,3-b]thiophène*** 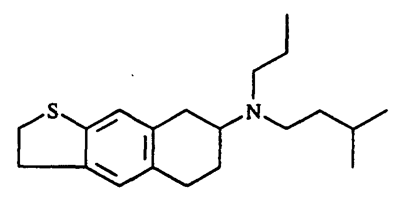 ***et son chlorhydrate.***

En procédant comme décrit dans l'exemple 4 mais en utilisant le chlorure de l'acide isovalérique à la place du chlorure propionyle on obtient le produit attendu, dont le chlorhydrate fond (MK) à 185 °C.

### Exemple 6

(3RS)-3-{N-[2-(cyclopropyl)éthyl] N-propylamino} 6-oxocyclopenta[g] 3,4-dihydro-2H-benzopyrane 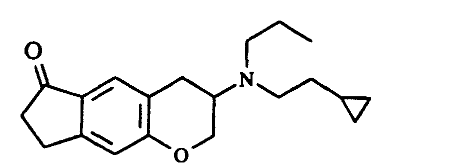 ***et son chlorhydrate*.**

En procédant comme décrit dans l'exemple 4 mais en utilisant le produit de la préparation 3 et en remplaçant le chlorure de propionyle par le chlorure de l'acide cyclopropylacétique, on obtient le produit attendu, dont le chlorhydrate fond (MK) à 87-94 °C.

### Exemple 7

***(3RS)-3-{N-[2-(p-hydroxyphenyl)éthyl] N-propylamino} 6-oxocyclopenta[g] 3,4-dihydro-2H-benzopyrane*** 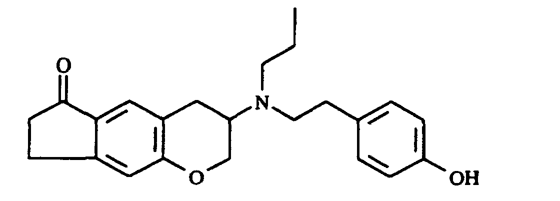 ***et son chlorhydrate.***

2 g (8,1 mmole) du composé obtenu à la préparation 3 et 2,03 g (10,1 mmole) de 1-bromo 2-(p-hydroxyphényl)éthane sont introduits dans une suspension de 2,6 g (24,3 mmole) de carbonate de sodium dans 50 ml d'acétone. On porte à reflux 24 h puis on rajoute de nouveau 2,03 g de 1-bromo 2-(p-hydroxyphényl)éthane et porte encore 48 h à reflux. Après évaporation à sec, on reprend par 100 ml de CH₂Cl₂ et lave par deux fois 30 ml d'eau puis sèche sur MgSO₄. Le résidu est chromatographié sur silice (éluant CH₂Cl₂/CH₃OH : 99/1) pour donner 1,37 g de base libre, transformée en chlorhydrate par addition d'une solution 2N d'éther chlorhydrique. On obtient 0,97 g du chlorhydrate attendu. PF(MK) : 122-128 °C. Rendement : 29 %

RMN 1H (DMSO d6/TMS) δ : 11,15 (massif échangeable par D₂O) ; 9,35 (1H,échangeable par D₂O) ; 7,5 (s,1H) ; 7,1 (d,2H) ; 7,05 (s,1H) ; 6,7 (d,2H) ; 4,7 et 4,5 (2m,2H) ; 4,0 (m,1H) ; 3,5 à 2,9 (m,10H) ; 2,6 (t,2H) ; 1,8 (m,2H) ; 0,9 (t,3H).

### Exemple 8

***(3RS)-3-{N-[(2E)but-2-ényl]N-propylamino}6-oxocyclopenta[g]3,4-dihydro-2H-benzo pyrane*** 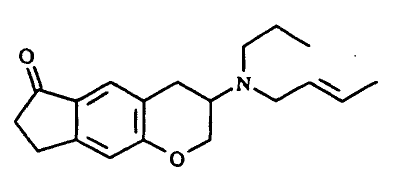 ***et son chlorhydrate***

En procédant comme décrit à l'exemple 7 mais en utilisant le 4-bromo but-2-ène sous forme trans à la place du 1-bromo 2-(p-hydroxyphényl)éthane, on obtient le produit attendu dont le chlorhydrate fond (MK) à 87-91 °C.

### Exemple 9

***(3RS)-3-{N-[2-((3H)-indol-2-on-4-yl)éthyl] N-propylamino} 6-oxocyclopenta[g] 3,4-dihydro-2H-benzopyrane.***

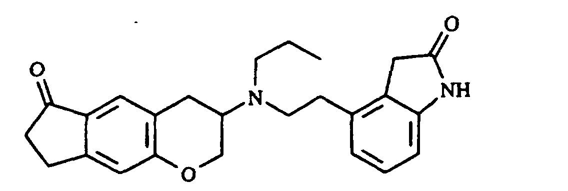

En procédant comme décrit à l'exemple 7 mais en utilisant le tosylate du 1-hydroxy 2-[(3H)-indol-2-on-4-yl]éthane à la place du 1-bromo 2-(p-hydroxyphényl)éthane, on obtient le produit attendu qui fond (MK) à 190-192 °C.

### Exemple 10

***(7RS)-7-{N-[3-(4-acétylaminophényl)propyl] N-propylamino} 2,3,5,6,7,8-hexahydronaphto [2,3-b]thiophène*** 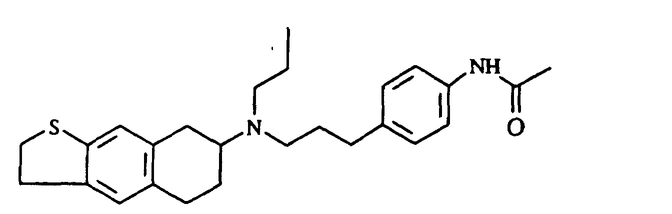 ***et son chlorhydrate***

En procédant comme décrit à l'exemple 7 mais en utilisant le produit de la préparation 4 et en remplaçant le 1-bromo 2-(p-hydroxyphényl)éthane par le 3-(4-acétylaminophényl) 1-iodo propane, on obtient le produit attendu dont le chlorhydrate fond(MK) à 130-135 °C.

### Exemple 11

***(7RS)-7-{N-(2-(4-acétylaminophényl)éthyl] N-propylamino} 2,3,5,6,7,8-hexahydronaphto [2,3-b]thiophène*** 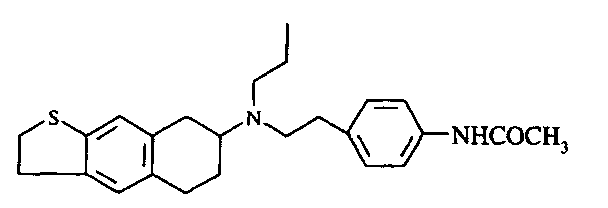 ***et son chlorhydrate***

En procédant comme décrit à l'exemple 7 mais en utilisant le produit de la préparation 4 et en remplaçant le 1-bromo 2-(p-hydroxyphényl)éthane par le 2-(4-acétylaminophényl) 1-iodo éthane, on obtient le produit attendu dont le chlorhydrate fond (MK) à 145-150 °C.

### Exemple 12

***(7RS)-7-{N-[3-(4-aminocarbonylphényl)propyl] N-propylamino} 2,3,5,6,7,8-hexahydro naphto[2,3-b]thiophène***

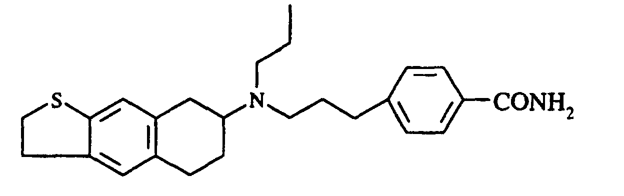

En procédant comme décrit à l'exemple 7 mais en utilisant le produit de la préparation 4 et en remplaçant le 1-bromo 2-(p-hydroxyphényl)éthane par le mésylate du 3-(4-aminocarbonylphényl)propan-1-ol, on obtient le produit attendu, qui fond (MK) à 122-124 °C.

### Exemple 13

***(3RS)-3-{N-[4-(4-bromobenzamido)butyl] N-propylamino} 6-oxocyclopenta [g] 3,4-dihydro-2H-benzopyrane*** 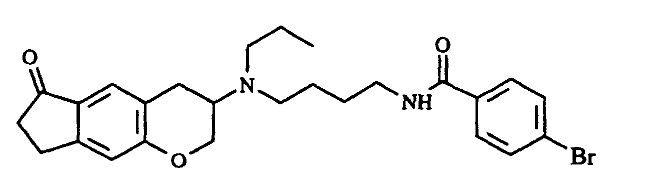 ***et son chlorhydrate***

Dans un mélange de 0,57 ml d'acide acétique et de 40 ml de 1,2-dichloroéthane, on introduit 2,45 g (10,0 mmole) du composé obtenu à la préparation 3 et 2,7 g (10,0 mmole) de 4-(4-bromo benzamido)butyraldéhyde. Tout en refroidissant à 10 °C, on ajoute par portion 3,2 g (15 mmole) d'acétoxyborohydrure de sodium, puis on abandonne 48 h à température ambiante. Après addition de 50 ml d'eau, on sépare les phases et réextrait deux fois la phase aqueuse par 50 ml de CH₂Cl₂. Les phases organiques réunies sont séchées sur MgSO₄, concentrées et purifiées sur silice en utilisant pour éluant un mélange CH₂Cl₂/CH₃OH 98/2. On obtient alors 3,6 g de produit attendu. Le chlorhydrate correspondant est préparé par addition d'une solution 1,8 N d'éther chlorhydrique. On obtient 3,74 g de chlorhydrate. (Rendement : 69,9 %) dont le point de fusion (MK) est de 110-115 °C.

### Exemple 14

***(7RS)-7-{N-[4-(4-bromobenzamido)butyl] N-propylamino} 2,3,5,6,7,8-hexahydronaphto [2,3-b]thiophène*** 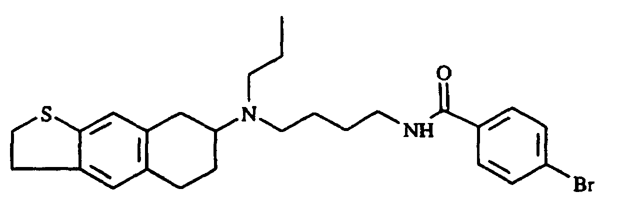 ***et son chlorhydrate***

En procédant comme décrit à l'exemple ci-dessus, mais en utilisant le produit de la préparation 4 à la place de celui de la préparation 3 , on obtient le produit désiré dont le chlorhydrate fond (MK) à 100-110 °C.

### Exemple 15

***(7RS)-7-(N-pentyl N-propylamino) 2,3,5,6,7,8-hexahydronaphto[2,3-b]thiophène*** 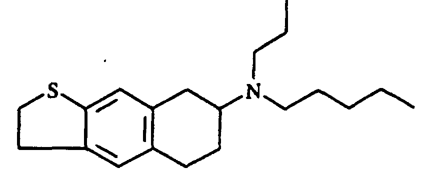 ***et son chlorhydrate***

En procédant comme décrit à l'exemple 13 mais en utilisant le produit de la préparation 4 et en remplaçant la 4-(4-bromobenzamido)butyraldéhyde par la valéraldéhyde, on obtient le produit désiré dont le chlorhydrate fond à 140-142 °C.

### Exemple 16

***(7RS)-7-{N-[3-(4-tert-butoxycarbonylaminophényl)propyl] N-propylamino} 2,3,5,6,7,8-hexahydronaphto[2,3-b]thiophène***

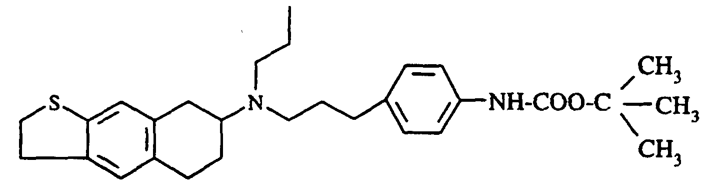

En procédant comme décrit à l'exemple 13 mais en utilisant le produit de la préparation 4 et en remplaçant la 4-(4-bromobenzamido)butyraldéhyde par la 3-(4-tert-butoxycarbonylamino phényl)propionaldéhyde, on obtient le produit désiré sous forme de meringue.

### Exemple 17

***(7RS)-7-{N-[3-(4-aminophényl)propyl] N-propylamino} 2,3,5,6,7,8-hexahydronaphto[2,3-b] thiophène***

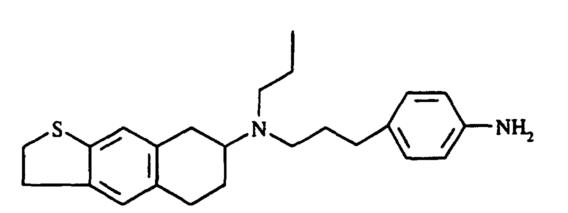

1,62 g du produit de l'exemple précédent (3,36 mmole) en solution dans 10 ml de chlorure de méthylène est traité, goutte à goutte, à température ambiante par 2 ml d'o-méthoxyphénylthiol, puis 10 ml d'acide trifluoro acétique. Après 3 h à température ambiante, le milieu réactionnel est concentré sous vide, le résidu est lavé par l'éther puis repris à l'eau et lavé une nouvelle fois à l'éther. La phase aqueuse est alcalinisée par de la soude normale et extraite par de l'éther. Après séchage de la phase organique, on obtient 0,86 g de produit attendu sous forme de meringue (Rendement : 67 %).

### Exemple 18

***(7RS)-7-{N-[3-(4-trifluoroacétylaminophényl)propyl]N-propylamino}2,3,5,6,7,8-hexahydro naphto [2,3-b]thiophène*** 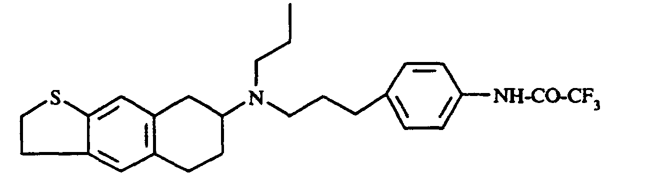 ***et son chlorhydrate***

0,43 g (1,13 mmole) du produit obtenu à l'exemple précédent est traité à 0°C, dans 11 ml de chlorure de méthylène par 0,18 ml d'anhydride trifluoroacétique. Après 30 minutes, on dilue par une solution saturée de bicarbonate de sodium et on extrait au chlorure de méthylène. Après lavage et séchage, on obtient le produit attendu, dont la totalité est transformée en chlorhydrate (0,37 g). PF (MK) : 113-120 °C.

### Exemple 19

***(7RS)-7-{N-[3-(4-méthylsulfonylaminophényl)propyl]N-propylamino}2,3,5,6,7,8-hexahydro naphto[2,3-b]thiophène*** 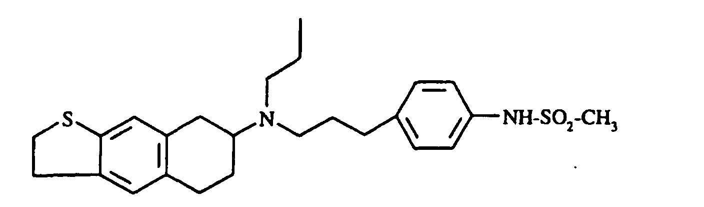 ***et son chlorhydrate***

0,43 g (1,13 mmole) du produit obtenu à l' exemple 17 mis en solution dans 11 ml de chlorure de méthylène est traité à 0 °C par 0,11 ml de chlorure de mésyle et 0,31 ml de triéthylamine. Après 1h à température ambiante, on dilue par une solution saturée de bicarbonate de sodium et extrait au chlorure de méthylène. Après lavage et séchage, on obtient le produit attendu, dont la totalité est transformée en chlorhydrate (0,15 g). PF (MK) : 137-140 °C.

### Exemple 20

***(7RS)-7-(N,N-dipropylamino) 1-oxo cyclopenta[g] 5,6,7,8-tétrahydronaphtalène***

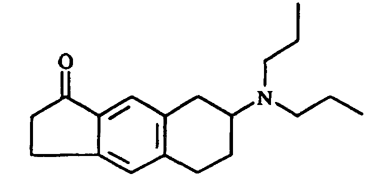

### 1 ère méthode

En utilisant la méthode décrite au stade 2 de l'exemple 1 mais en partant du produit de la préparation 5 au lieu du produit de la préparation 1, on obtient après séparation par HPLC (colonne KC18 ; phase mobile : H₂O,CH₃CN,TFA/800,200,1) et basification, le produit attendu dont le point de fusion (MK) est de 66-69 °C.

### 2ème méthode

### Stade A : Ester de méthyle de l'acide trans 3-[2-(N,N-dipropylamino)1,2,3,4-tétrahydronapht-6-yl]prop-2-énoïque

On dissout 3,1 g (0,01 mole) de 6-bromo 2-(N,N-dipropylamino) 1,2,3,4-tétrahydronaphtalène dans 50 ml de diméthylformamide. A cette solution, on ajoute successivement 0,94 g (0,011 mole) d'acrylate de méthyle, 22,4 mg (0,1 mmole) d'acétate de palladium, 121 mg (0,4 mmole) de tri-orthotolylphosphine et 1,6 ml (0,011 mole) de triéthylamine. On porte 3 h 30, sous agitation, à 125-130 °C. On refroidit et verse dans 700 ml d'eau, on extrait à l'acétate d'éthyle, lave par une solution de LiCl. Après passage acide-base, on isole 0,9 g de produit attendu.

### Stade B : Ester de méthyle de l'acide 3-[2-(N,N-dipropylamino) 1,2,3,4-tétrahydronapht-6-yl]propionique

Dans 35 ml de méthanol, on introduit 2,1 g (6,6 mmole) de produit préparé au stade précédent et 0,2 g d'oxyde de platine. On hydrogène à pression atmosphérique et à température ambiante pendant 18 h. On filtre et concentre pour isoler 2 g de produit attendu. Rendement : 95 %.

### Stade C : Acide 3-[2-(N,N-dipropylamino) 1,2,3,4-tétrahydronapht-6-yl]propionique

On place dans un bicol 2,5 g (7,8 mmole) du produit obtenu au stade précédent, 20 ml de méthanol et 8 ml de soude normale. On laisse sous agitation, à température ambiante pendant 18 h. On acidifie par 8 ml d'HCl 1N et concentre. On reprend par de l'acétonitrile, filtre un insoluble et concentre pour obtenir après séchage 2 g du produit attendu sous forme d'huile visqueuse. Rendement : 85 %.

### Stade D : produit titre

On porte 20 g d'acide polyphosphonique à 75 °C. On ajoute en une seule fois 1,8 g (6 mmole) de l'acide préparé au stade C précédent, et conserve à cette température pendant 1 h. On laisse refroidir partiellement et ajoute de la glace. On basifie à la lessive de soude en présence d'acétate d'éthyle. On sèche et concentre pour obtenir 0,8 g d'un résidu qu'on purifie par HPLC dans des conditions identiques à celles utilisées dans la première méthode. Après basification, on obtient 0,2 g de produit attendu qui fond (MK) à 68-70 °C.

RMN 300 MHz (CDCl₃/TMS)
7,5(s,1H) ; 7,2(s,1H) ; 3,1(t,2H) ; 3,1 à 2,7 (m,5H) ; 2,7(t,2H) ; 2,5(m,4H) ; 2,1 à 1,6(m,2H) ; 1,5(m,4H) ; 0,9(t,6H).

### Exemple 21

### (7RS)-7-(N,N-dipropylamino)1-oxo 2,3,5,6,7,8-hexahydronaphto[2,3-b]thiophéne.

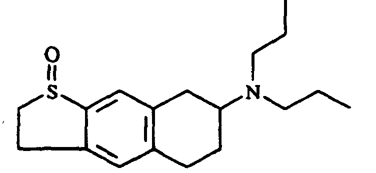

1,96 g (6 mmole) du produit obtenu à l'exemple 4, sous forme de chlorhydrate en solution dans un mélange de 38 ml d'eau et 6 ml d'acide chlorhydrique 1 N est traité par 0,85 ml d'eau oxygénée à 30 %. Le mélange est chauffé 30 minutes à 80 °C. Après refroidissement , le mélange réactionnel est alcalinisé et extrait à l'éther pour donner après évaporation 1,81 g du résidu qui est purifié par flash chromatographie sur colonne de silice (éluant CH₂Cl₂-MeOH/95-5) pour donner 1,28 g de produit attendu sous forme huileuse. Rendement : 70 %.

RMN 200 MHz (CDCl₃/TMS) 7,65(s,1H) 7,3(s,1H) ; 3,8 à 3,5 (m,2H) ; 3,5 à 2,8(m,11H) ; 2,35(m,1H) ; 2,0 à 1,7(m,5H) ; 0,85(t,6H)

### Exemple 22

***(7RS)-7-(N,N-dipropylamino) 5,6,7,8-tétrahydronaphto[2,3-b] thiophène*** 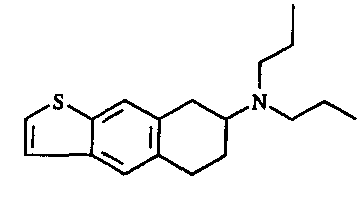 ***et son chlorhydrate***

1 g du produit préparé à l'exemple précédent est traité à température ambiante par 20 ml d'une solution 2,5 N d'éther chlorhydrique pendant 6 h. Après alcalinisation, le milieu réactionnel est chromatographié pour donner 0,3 g de produit attendu, dont le chlorhydrate fond à 212-215 °C (MK).
RMN (200 MHz CDCl₃)
12,5 (massif échangeable par D₂O) ; 7,65 (s,1H) 7,55(s,1H) ; 7,4(d,1H) ; 7,25(d,1H) ; 3,75(m,1H) ; 3,5 à 2.9(m,8H) ; 2,65(m,1H) ; 2,3 à 1,9(m,5H) ; 1,05(t,6H).

### Exemple 23

### Etude pharmacologique

### ETUDES DE LA LIAISON DES COMPOSES DE L'INVENTION AUX RECEPTEURS D₂ ET D₃

Les résultats sont exprimés sous la forme de pKᵢ (pKᵢ = - logKᵢ).

La valeur de Kᵢ est dérivée de la formule Kᵢ = IC₅₀/(1 + L/KD) où L est la concentration de [¹²⁵I]-Iodosulpride utilisée dans l'expérience et Kd sa constante de dissociation.

L'IC₅₀ représentant la concentration donnant 50 % d'inhibition de la liaison du radioligand est calculée par régression non linéaire (méthode Simplex).

Les produits de la présente invention montrent, pour le récepteur D₃, des affinités dont le pKᵢ est compris entre 7 et 9.

De plus, tous les produits de l'invention présentent des affinités pour le récepteur D₂ de 10 à 80 fois inférieures à celles présentées pour le récepteur D₃.
Les produits de la présente invention se comportent donc comme des ligands beaucoup plus sélectifs que les aminotétralines de référence (+) AJ 76 et (+) UH 232, dont les sélectivités ne sont respectivement que de 2 et 5.

## Revendications

1. Les amines hétérocycliques tertiaires de formule I : 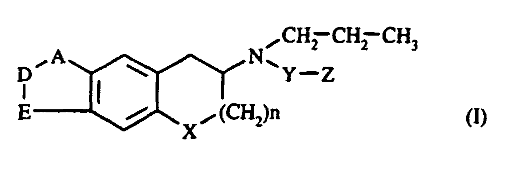 dans laquelle :
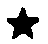 **-A-D-E-** représente 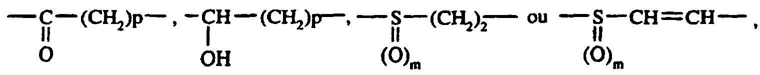 dans lesquels
p représente 2 ou 3 et
m représente zéro, 1 ou 2 ;
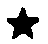 **X** représente :
un groupe -CH₂- et de plus,
lorsque -A-D-E- représente 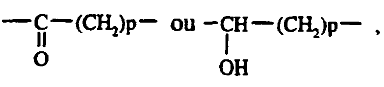 X peut aussi représenter un atome d'oxygène ;
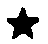 **n** représente :
zéro ou 1 lorsque X représente le groupe CH₂, et
uniquement 1 lorsque X représente un atome d'oxygène ;
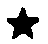 **Y** représente une liaison simple ou un groupe -CH₂-, et
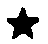 **Z** représente :
un radical alkyle, alcényle ou alcynyle chacun contenant jusqu'à 10 atomes de carbone inclus, en chaîne droite ou ramifiée, et chacun éventuellement substitué par un ou plusieurs radicaux cycloalkyle ayant de 3 à 7 atomes de carbone inclus, ou
un radical choisi parmi 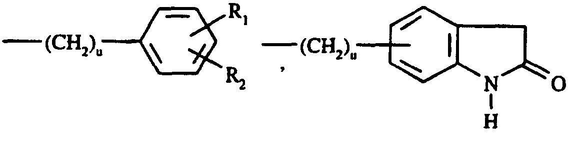 et 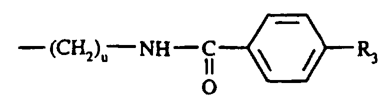 dans lesquels
u représente un nombre entier de 1 à 6 inclus ;
R₁ représente un atome d'hydrogène ou d'halogène, un radical hydroxy ou un radical alkyl ou alkoxy contenant chacun de 1 à 6 atomes de carbone inclus en chaîne droite ou ramifiée ;
R₂ représente un atome d'halogène, un radical hydroxy, un radical alkyle ou alkoxy contenant chacun de 1 à 6 atomes de carbone inclus en chaîne droite ou ramifiée, un radical phényle, ou un groupement de formule NHCOO-C(CH₃)₃, NH₂, NH-COCH₃, NHCOCF₃ , NHSO₂CH₃ et 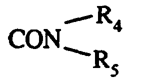 dans le quel R₄ et R₅, identiques
ou différents, représentent chacun un atome d'hydrogène ou un radical (C₁-C₆)alkyle en chaine droite ou ramifiée ;
et
R₃ représente un atome d'halogène ou un radical hydroxy, (C₁-C₅)alkoxy en chaine droite ou ramifiée, trifluorométhyle, cyano, phényle, p-aminophényle ou p-acétylphényle;
sous forme racémique ou d'isomères optiques,
et leurs sels d'addition avec un acide pharmaceutiquement acceptable.

2. Un composé de la revendication 1 qui est le (7RS)-7-(N,N-dipropylamino) 2,3,5,6,7,8-hexahydronaphto[2,3-b]thiophène et son chlorhydrate.

3. Un composé de la revendication 1 qui est le (3RS)- 3-{N-[2-(p-hydroxyphényl)éthyl] N-propylamino} 6-oxocyclopenta[g]3,4-dihydro-2H-benzopyrane et son chlorhydrate

4. Un composé de la revendication 1 qui est le (7RS)-7-(N-pentyl N-propylamino) 2,3,5,6,7,8-hexahydronaphto[2,3-b]thiophène

5. Un composé de la revendication 1 qui est le (7RS)-7-(N,N-dipropylamino) 1-oxo cyclopenta[g] 5,6,7,8-tétrahydronaphtalène.

6. Le procédé de préparation des composés de la revendication 1 **caractérisé en ce que**
∗ l'on fait réagir :
. une amine secondaire de formule II : 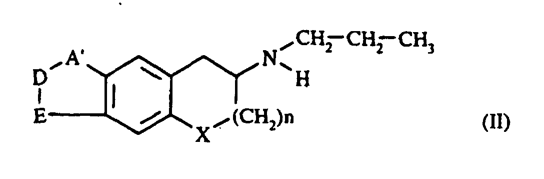 dans laquelle :
X et n ont les significations définies dans la revendication 1 et
A'-D-E représente : (CH₂)₃, (CH₂)₄, -S-(CH₂)₂- ou -S-CH=CH-,
. avec un composé de formule III :
G-Y-Z (III)
dans laquelle :
Y et Z ont les significations définies dans la revendication 1 et
G représente un atome d'halogène, un radical mésyloxy ou un radical tosyloxy
∗ et l'on oxyde les composés de formule IV ainsi obtenus : 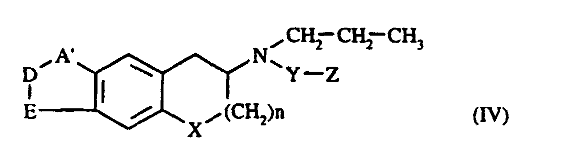 dans laquelle :
-A'-D-E, X, n, Y et Z ont les significations précédemment définies au moyen :
∼ soit du réactif de Jones, ou de 2,3-dichloro-5,6-dicyano-1,4-benzoquinone dans acide acétique et eau, dans le cas où -A'-D-E- prend les valeurs (CH₂)₃ et (CH₂)₄,
∼ soit de l'eau oxygénée, dans le cas où -A'-D-E- prend les valeurs -S-(CH₂)₂- ou - S-CH=CH-,
∗ pour obtenir les composés de formule Ia : 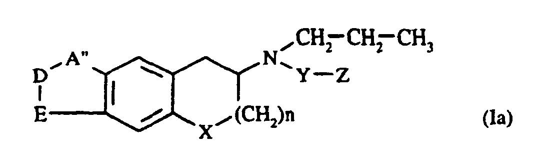 dans laquelle :
X, n, Y et Z ont les significations précédemment définies et
A"-D-E représente un enchaînement choisi parmi : 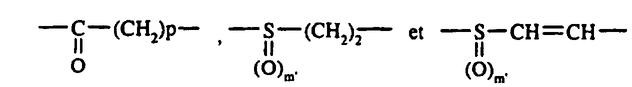 dans lesquels m' représente 1 ou 2 et p est tel que défini dans la revendication 1;
∗ et dans le cas où A"-D-E représente 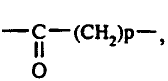 on réduit les composés de formule Ia correspondants [c'est-à-dire les composés répondant plus spécifiquement à la formule I'a : 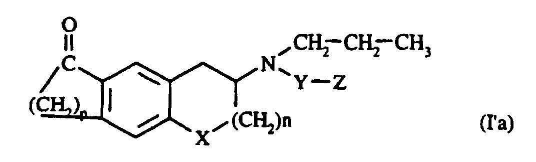 dans laquelle p, X, n, Y et Z ont les significations précédemment définies]
∗ pour obtenir les composés de formule Ib : 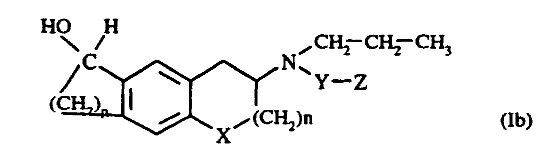 dans laquelle p, X, n, Y et Z ont les significations précédemment définies ;
et si on le désire, on transforme les composés de formule IV, Ia et Ib (qui forment l'ensemble des composés de formule I) en leurs sels d'addition avec des acides pharmaceutiquement acceptables.

7. Le procédé de préparation des composés de formule I' : 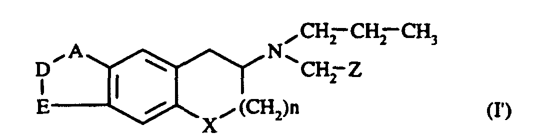 dans laquelle A-D-E, X, n et Z ont les significations définies dans la revendication 1,
- **caractérisé en ce que** l'on fait réagir l'amine secondaire de formule II définie dans la revendication 6,
avec :
. soit un composé de formule V : 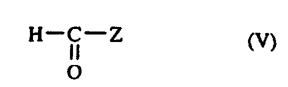 dans laquelle Z a la signification précédemment définie,
en opérant en milieu réducteur,
. soit un composé de formule VI : 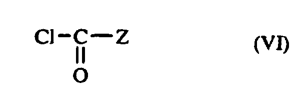 dans laquelle Z a la signification précédemment définie,
et l'on réduit le composé obtenu de formule VII : 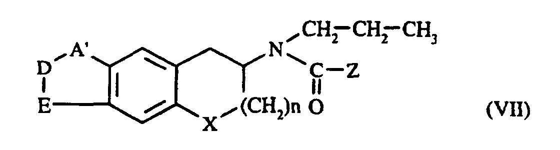 dans laquelle A'-D-E, X, n et Z ont les significations définies dans la revendication 6,
- afin d'obtenir, quelle que soit la voie réactionnelle utilisée, un composé de formule VIII : 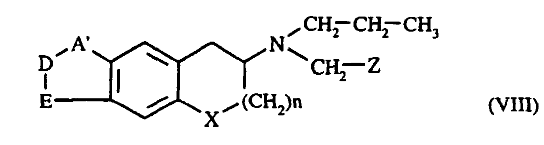 dans laquelle A'-D-E, X, n et Z ont les significations précédemment définies ;
- lequel composé de formule VIII est alors transformé par oxydation au moyen :
. soit du réactif de Jones, ou de 2,3-dichloro-5,6-dicyano-1,4-benzoquinone dans acide acétique et eau, dans le cas où A'-D-E prend les valeurs (CH₂)₃ et (CH₂)₄,
. soit de l'eau oxygénée, dans le cas où -A'-D-E- prend les valeurs -S-(CH₂)₂- ou -S-CH=CH,
- pour obtenir les composés de formule I"a : 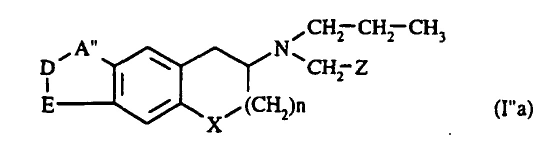 dans laquelle A"-D-E, X, n et Z ont les significations définies dans la revendication 6,
- et dans le cas où A"-D-E représente 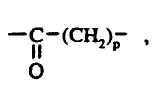 on réduit les composés de formule I"a correspondants [c'est-à-dire les composés correspondant plus spécifiquement à la formule I'''a : 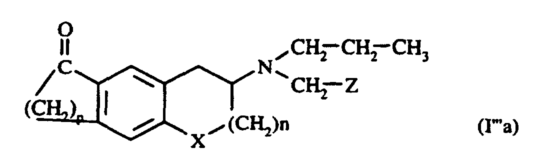 [dans laquelle p, X, n et Z ont les significations définies dans la revendication 1]
pour obtenir les composés de formule I'b : 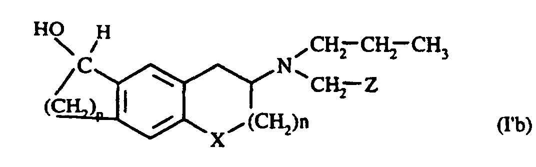 dans laquelle p, X, n et Z ont les significations précédemment définies.

8. Le procédé de préparation des composés de formule I" : 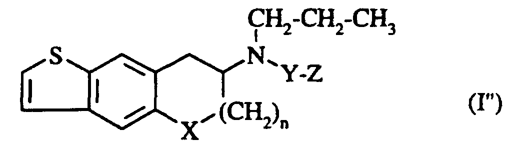 dans laquelle X, n, Y et Z ont les significations définies dans la revendication 1,
**caractérisé en ce que** l'on oxyde au moyen d'eau oxygénée les composés de formule IX : 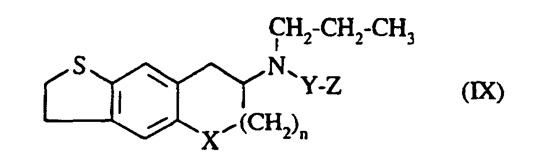 dans laquelle X, n, Y et Z ont les significations précédemment définies,
pour obtenir les composés de formule X : 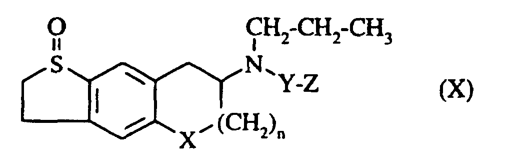 [dans laquelle X, n, Y et Z ont les significations précédemment définies]
que l'on transforme en composés de formule I" au moyen d'acide chlorhydrique et d'éther.

9. Le procédé de préparation des composés de formule I''' : 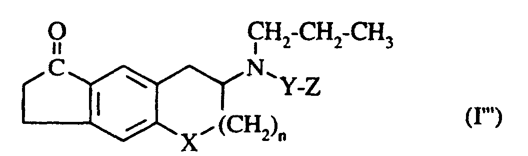 dans laquelle X, n, Y et Z ont les significations définies dans la revendication 1,
**caractérisé en ce que** :
l'on traite les composés de formule XI : 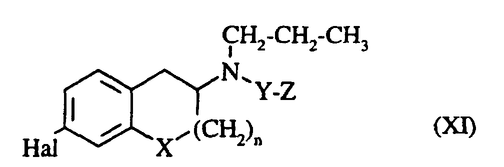 dans laquelle X, n, Y et Z ont les significations précédemment définies, et Hal représente un atome de brome ou d'iode,
au moyen d'acrylate de méthyle dans le diméthylformamide, en présence d'acétate de palladium, de tri-o-tolylphosphine et de triéthylamine,
pour donner les composés de formule XII : 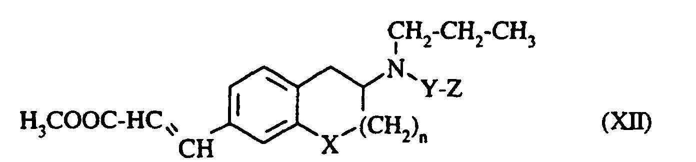 dans laquelle X, n, Y et Z ont les significations précédemment définies,
que l'on réduit par l'hydrogène en présence de platine sur charbon pour conduire aux composés de formule XIII : 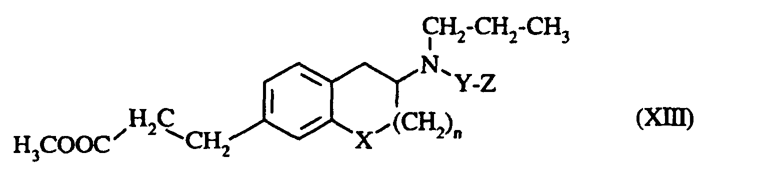 dans laquelle X, n, Y et Z ont les significations précédemment définies,
que l'on traite par la soude pour donner les composés de formule XIV : 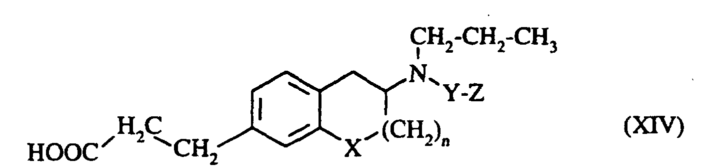 dans laquelle X, n, Y et Z ont les significations précédemment définies,
lesquels sont cyclisés par action de l'anhydride phosphorique pour obtenir les composés de formule I''' précédemment définie.

10. Les compositions pharmaceutiques utilisables dans le traitement de la maladie de Parkinson, des troubles de la mémoire, des troubles liés à l'abus de drogue, de la dépression et des états psychotiques, contenant comme principe actif un composé selon une quelconque des revendications 1 à 5, avec un ou plusieurs excipients pharmaceutiques appropriés.

## Patentansprüche

1. Heterocyclische tertiäre Amine der Formel I: 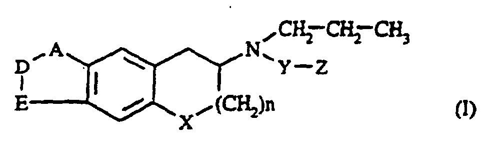 in der:
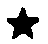 **-A-D-E-** 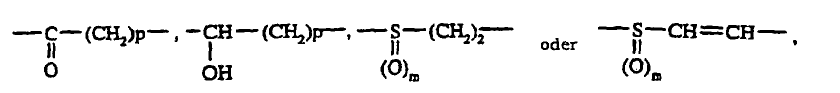 bedeutet, worin
p 2 oder 3 und
m Null, 1 oder 2 darstellen;
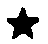 **X**:
eine Gruppe -CH₂- bedeutet und
wenn -A-D-E- 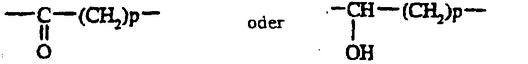 darstellt,
X auch ein Sauerstoffatom bedeuten kann;
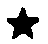 **n**:
Null oder 1 bedeutet, wenn X die Gruppe CH₂ darstellt, und
lediglich 1 bedeutet, wenn X ein Sauerstoffatom darstellt;
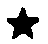 Y eine Einfachbindung oder eine Gruppe -CH₂- bedeutet, und
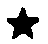 **Z**:
eine Alkyl-, Alkenyl- oder Alkinyl-Gruppe, die jeweils bis zu 10 Kohlenstoffatomen einschließlich in gerader oder verzweigter Kette aufweisen und jeweils gegebenenfalls durch ein oder mehrere Cycloalkylgruppen mit 3 bis 7 Kohlenstoffatomen einschließlich substituiert sein können, oder
eine Gruppe ausgewählt aus 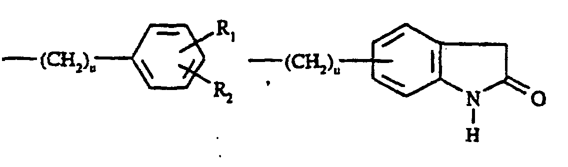 und 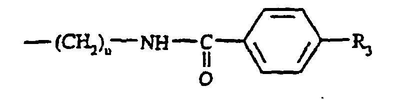 bedeutet, in denen
u eine ganze Zahl mit einem Wert von 1 bis 6 einschießlich;
R₁ ein Wasserstoffatom, ein Halogenatom, eine Hydroxygruppe oder eine Alkyl- oder Alkoxy-Gruppe mit jeweils 1 bis 6 Kohlenstoffatomen einschließlich in gerader oder verzweigter Kette;
R₂ ein Halogenatom, eine Hydroxygruppe, eine Alkyl- oder Alkoxy-Gruppe, die jeweils 1 bis 6 Kohlenstoffatome einschließlich in gerader oder verzweigter Kette enthalten, eine Phenylgruppe oder eine Gruppe der Formel NHCOO-C(CH₃)₃, NH₂, NH-COCH₃, NHCOCF₃, NHSO₂CH₃ und 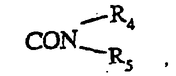 worin R₄ und R₅, die gleichartig oder verschieden sind, jeweils ein Wasserstoffatom oder eine (C₁-C₆)-Alkylgruppe in gerader oder verzweigter Kette darstellen;
und
R₃ ein Halogenatom oder eine Hydroxygruppe, (C₁-C₅)-Alkoxygruppe in gerader oder verzweigter Gruppe, Trifluormethylgruppe, Cyanogruppe, Phenylgruppe, p-Aminophenylgruppe oder p-Acetylphenylgruppe bedeuten;
in racemischer Form oder in Form der optischen Isomeren,
und deren Additionssalze mit einer pharmazeutisch annehmbaren Säure.

2. Verbindung nach Anspruch 1, nämlich (7RS)-7-(N,N-Dipropylamino)-2,3,5,6,7,8-hexahydronaphtho[2,3-b]thiophen und dessen Hydrochlorid.

3. Verbindung nach Anspruch 1, nämlich (3RS)-3-{N-[2-(p-Hydroxyphenyl)-ethyl]-N-propylamino}-6-oxocyclopenta[g]3,4-dihydro-2H-benzopyran und dessen Hydrochlorid.

4. Verbindung nach Anspruch 1, nämlich (7RS)-7-(N-Pentyl-N-propylamino)-2,3,5,6,7,8-hexahydronaphtho[2,3-b]thiophen.

5. Verbindung nach Anspruch 1, nämlich (7RS)-7-(N,N-Dipropylamino)-1-oxocyclopenta[g]-5,6,7,8-tetrahydronaphthalin.

6. Verfahren zur Herstellung der Verbindungen nach Anspruch 1, **dadurch gekennzeichnet, daß** man
∗ :
. ein sekundäres Amin der Formel II: 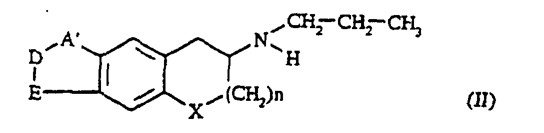 in der:
X und n die in Anspruch 1 angegebenen Bedeutungen besitzen und
A'-D-E (CH₂)₃, (CH₂)₄, -S-(CH₂)₂ oder -S-CH=CH- darstellt,
. mit einer Verbindung der Formel III umsetzt:
G-Y-Z (III)
in der:
Y und Z die in Anspruch 1 angebenen Bedeutungen besitzen und
G ein Halogenatom, eine Mesyloxygruppe oder eine Tosyloxygruppe darstellt,
∗ und die in dieser Weise erhaltenen Verbindungen der Formel IV: 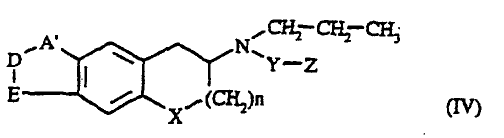 in der:
-A'-D-E, X, n, Y und Z die oben angegebenen Bedeutungen besitzen:
∼ entweder mit dem Jones-Reagens oder mit 2,3-Dichlor-5,6-dicyano-1,4-benzochinon in Essigsäure und Wasser dann, wenn -A'-D-E- die Bedeutungen (CH₂)₃ und (CH₂)₄ aufweist,
∼ oder mit Wasserstoffperoxid dann, wenn -A'-D-E die Bedeutungen -S-(CH₂)₂- oder -S-CH=CH- aufweist, oxidiert,
∗ zur Bildung der Verbindungen der Formel Ia: 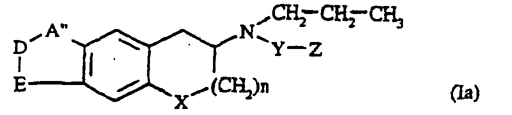 in der:
X, n, Y und Z die oben angegebenen Bedeutungen besitzen und
A"-D-E eine Kette ausgewählt aus: 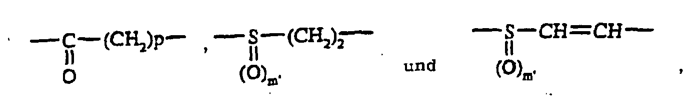 worin m' 1 oder 2 bedeutet und p die in Anspruch 1 angegebenen Bedeutungen besitzt, darstellt;
∗ und dann, wenn A"-D-E 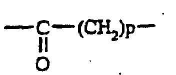 bedeutet, die entsprechenden Verbindungen der Formel Ia [das heißt die Verbindungen, die genauer der Formel I'a entsprechen: 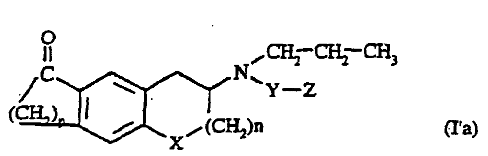 in der p, X, n, Y und Z die oben angegebenen Bedeutungen besitzen] reduziert,
∗ zur Bildung der Verbindungen der Formel Ib: 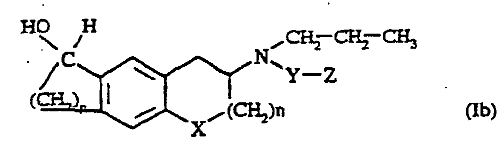 in der p, X, n, Y und Z die oben angegebenen Bedeutungen besitzen; und man gewünschtenfalls die Verbindungen der Formel IV, Ia und Ib (welche gemeinsam die Verbindungen der Formel I darstellen) in ihre Additionssalze mit pharmazeutisch annehmbaren Säuren überführt.

7. Verfahren zur Herstellung der Verbindungen der Formel I': 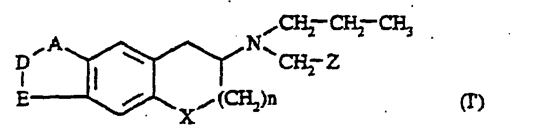 in der A-D-E, X, n und Z die in Anspruch 1 angegebenen Bedeutungen besitzen,
- **dadurch gekennzeichnet, daß** man das in Anspruch 6 definierte sekundäre Amin der Formel II
mit:
. entweder einer Verbindung der Formel V: 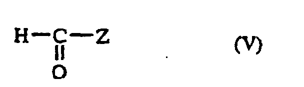 in der Z die oben angegebene Bedeutung besitzt,
wobei man in einem reduzierenden Medium arbeitet,
. oder mit einer Verbindung der Formel VI: 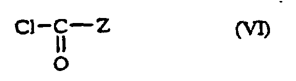 in der Z die oben angegebene Bedeutung besitzt, umsetzt,
und die erhaltene Verbindung der Formel VII: 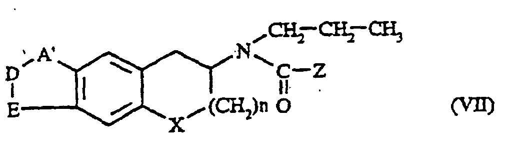 in der A'-D-E, X, n und Z die in Anspruch 6 angegebenen Bedeutungen besitzen, reduziert,
- so daß man unabhängig von dem angewandten Reaktionsweg eine Verbindung der Formel VIII erhält: 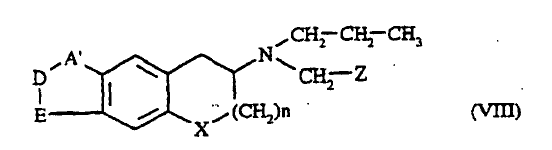 in der A'-D-E, X, n und Z die oben angegebenen Bedeutungen besitzen;
- welche Verbindung der Formel VIII man anschließend durch Oxidation mit:
. entweder dem Jones-Reagens oder mit 2,3-Dichlor-5,6-dicyano-1,4-benzochinon in Essigsäure und Wasser dann, wenn A'-D-E die Bedeutungen (CH₂)₃ und (CH₂)₄ besitzt,
. oder mit Wasserstoffperoxid dann, wenn -A'-D-E- die Bedeutungen -S(CH₂)₂- oder -S-CH=CH besitzt, umwandelt,
- zur Bildung der Verbindungen der Formel I"a: 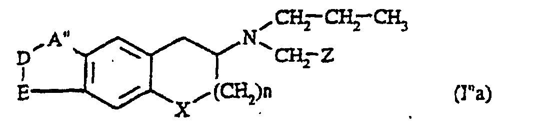 in der A"-D-E, X, n und Z die in Anspruch 6 angegebenen Bedeutungen besitzen,
- und dann, wenn A"-D-E 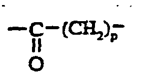 bedeutet, man die entsprechenden Verbindungen der Formel I"a [das heißt die Verbindungen, die genauer der Formel I'''a entsprechen: 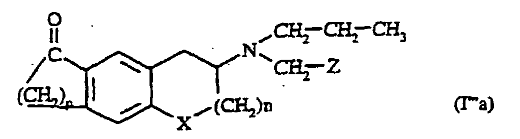 in der p, X, n und Z die Anspruch 1 angegebenen Bedeutungen besitzen] reduziert,
zur Bildung der Verbindungen der Formel I'b: 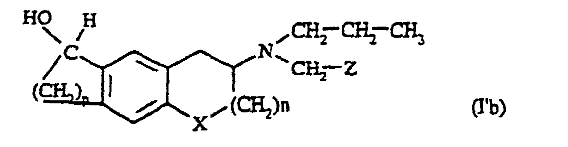 in der p, X, n und Z die oben angegebenen Bedeutungen besitzen.

8. Verfahren zur Herstellung der Verbindungen der Formel I": 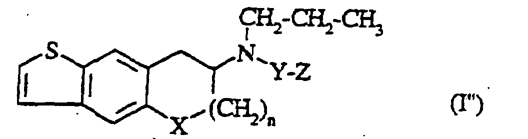 in der X, n, Y und Z die in Anspruch 1 angegebenen Bedeutungen besitzen,
**dadurch gekennzeichnet, daß** man die Verbindungen der Formel IX: 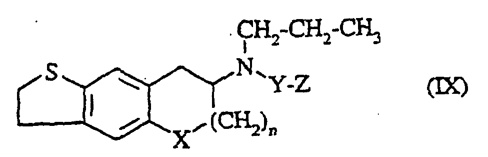 in der X, n, Y und Z die oben angegebenen Bedeutungen besitzen, mit Wasserstoffperoxid oxidiert,
zur Bildung der Verbindungen der Formel X: 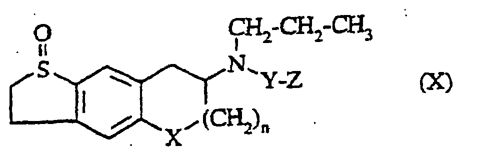 [in X, n, Y und Z die oben angegebenen Bedeutungen besitzen]
welche man mit Hilfe von Chlorwasserstoffsäure und Ether in die Verbindungen der Formel I" umwandelt.

9. Verfahren zur Herstellung der Verbindungen der Formel I''': 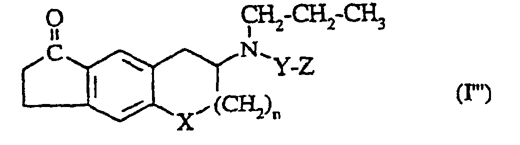 in der X, n, Y und Z die in Anspruch 1 angegebenen Bedeutungen besitzen,
**dadurch gekennzeichnet, daß** man
die Verbindungen der Formel XI: 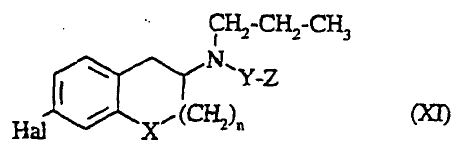 in der X, n, Y und Z die oben angegebenen Bedeutungen besitzen, und Hal ein Brom- oder Iod-Atom darstellt,
mit Methylacrylat in Dimethylformamid in Gegenwart von Palladiumacetat, Trio-tolylphosphin und Triethylamin behandelt,
zur Bildung der Verbindungen der Formel XII: 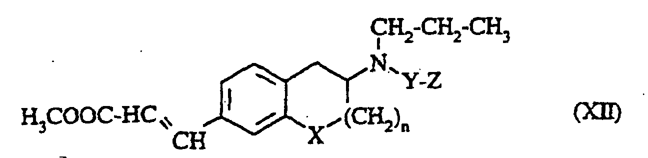 in der X, n, Y, Z die oben angegebenen Bedeutungen besitzen,
welche man mit Wasserstoff in Gegenwart von Platin-auf-Kohlenstoff reduziert zur Bildung der Verbindungen der Formel XIII: 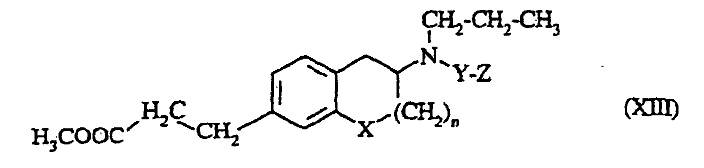 in der X, n, Y und Z die oben angegebenen Bedeutungen besitzen,
welche man mit Natriumhydroxid behandelt zur Bildung der Verbindungen der Formel XIV: 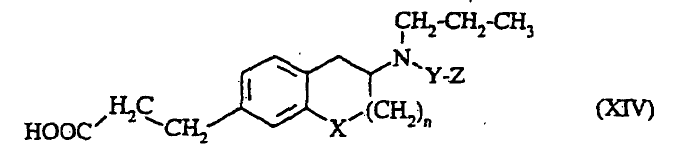 in der X, n, Y und Z die oben angegebenen Bedeutungen besitzen,
welche man durch Einwirkung von Phosphorsäureanhydrid cyclisiert zur Bildung der oben definierten Verbindungen der Formel I'''.

10. Pharmazeutische Zubereitungen für die Behandlung der Parkinsonschen Krankheit, von Gedächtnisstörungen, Störungen, die mit dem Drogenmißbrauch verknüpft sind, von Depressionen und psychotischen Zuständen, enthaltend als Wirkstoff eine Verbindung nach einem der Ansprüche 1 bis 5, zusammen mit einem oder mehreren pharmazeutisch annehmbaren Trägermaterialien.

## Claims

1. Heterocyclic tertiary amines of formula I : 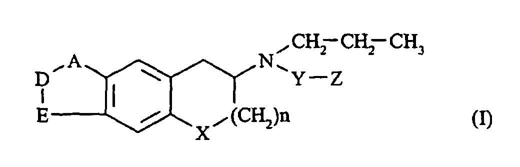 wherein :
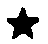 **-A-D-E-** represents 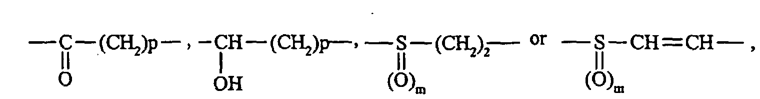 wherein
p represents 2 or 3 and
m represents zero, 1 or 2 ;
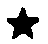 **X** represents :
a -CH₂- group and also,
when -A-D-E- represents 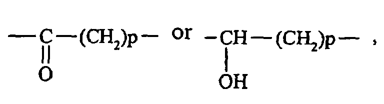 X may also represent an oxygen atom ;
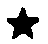 **n** represents:
zero or 1 when X represents a CH₂ group and
solely 1 when X represents an oxygen atom ;
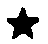 **Y** represents a single bond or a -CH₂- group, and
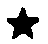 **Z** represents :
an alkyl, alkenyl or alkynyl radical, each containing up to 10 carbon atoms inclusive in straight or branched chain and each optionally substituted by one or more cycloalkyl radicals having from 3 to 7 carbon atoms inclusive, or
a radical selected from 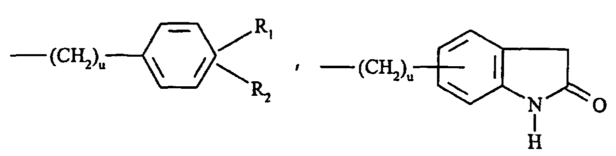 and 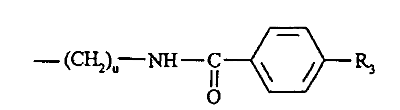 wherein
u represents an integer of from 1 to 6 inclusive ;
R₁ represents a hydrogen or halogen atom, a hydroxy radical or an alkyl or alkoxy radical each containing from 1 to 6 carbon atoms inclusive in straight or branched chain ;
R₂ represents a halogen atom, a hydroxy radical, an alkyl or alkoxy radical each containing from 1 to 6 carbon atoms inclusive in straight or branched chain, a phenyl radical or a group of the formula NHCOO-C(CH₃)₃, NH₂, NH-COCH₃, NHCOCF₃, NHSO₂CH₃ or 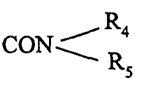 wherein R₄ and R₅, which are identical or different, each represents a hydrogen atom or a straight-chain or branched (C₁-C₆)alkyl radical ; and
R₃ represents a halogen atom or a hydroxy, straight-chain or branched (C₁-C₅)alkoxy, trifluoromethyl, cyano, phenyl, p-aminophenyl or p-acetylphenyl radical ;
in racemic form or in the form of optical isomers,
and the addition salts thereof with a pharmaceutically acceptable acid.

2. A compound of claim 1 which is (7RS)-7-(N,N-dipropylamino)-2,3,5,6,7,8-hexahydronaphtho[2,3-b]thiophene and its hydrochloride.

3. A compound of claim 1 which is (3RS)-3-{N-[2-(p-hydroxyphenyl)ethyl]-N-propylamino}-6-oxocyclopenta[g]3,4-dihydro-2H-benzopyran and its hydrochloride.

4. A compound of claim 1 which is (7RS)-7-(N-pentyl-N-propylamino)-2,3,5,6,7,8-hexahydronaphtho[2,3-b]thiophene.

5. A compound of claim 1 which is (7RS)-7-(N,N-dipropylamino)-1-oxocyclopenta[g]5,6,7,8-tetrahydronaphthalene.

6. A process for the preparation of the compounds of claim 1, **characterised in that**
∗
. a secondary amine of formula II : 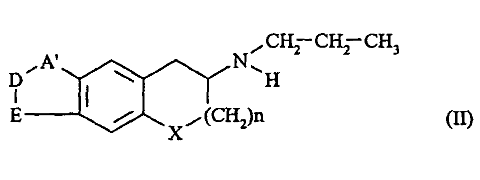 wherein
X and n are as defined in claim 1 and
A'-D-E represents: (CH₂)₃, (CH₂)₄, -S-(CH₂)₂- or -S-CH=CH-,
. is reacted with a compound of formula III :
G-Y-Z (III)
wherein :
Y and Z are as defined in claim 1 and
G represents a halogen atom, a mesyloxy radical or a tosyloxy radical
∗ and the compounds of formula IV so obtained : 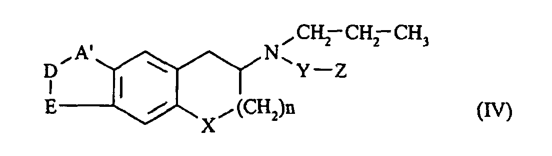 wherein :
-A'-D-E, X, n, Y and Z are as defined above are oxidized by means :
∼ either of Jones reagent, or of 2,3-dichloro-5,6-dicyano-1,4-benzoquinone in acetic acid and water, when -A'-D-E- represents (CH₂)₃ or (CH₂)₄,
∼ or of hydrogen peroxide when -A'-D-E- represents -S-(CH₂)₂- or -S-CH=CH-,
∗ to obtain the compounds of formula Ia : 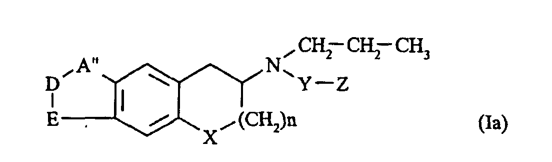 wherein :
X, n, Y and Z are as defined above and
A"-D-E represents a chain selected from: 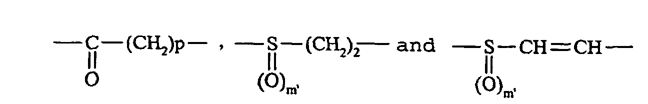 wherein m' represents 1 or 2 and p is as defined in claim 1;
∗ and, when A"-D-E represents 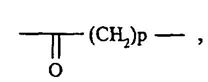 the corresponding compounds of formula Ia [that is to say, the compounds corresponding more specifically to formula I'a : 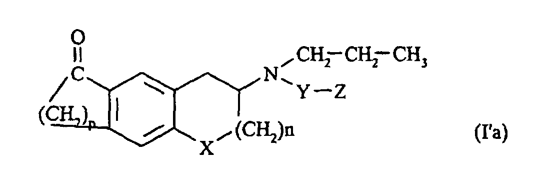 wherein p, X, n, Y and Z are as defined above] are reduced
∗ to obtain the compounds of formula Ib : 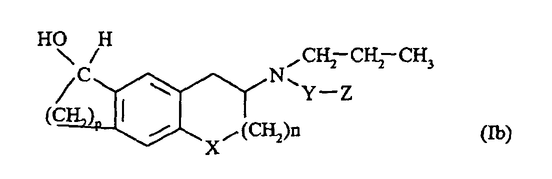
wherein p, X, n, Y and Z are as defined above,
and, if desired, the compounds of formula IV, Ia and Ib (which form the totality of the compounds of formula I) are converted into their addition salts with pharmaceutically acceptable acids.

7. A process for the preparation of compounds of formula I' : 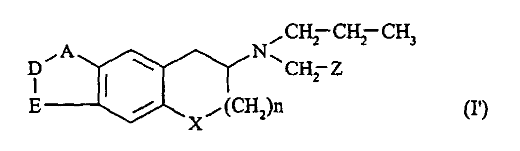 wherein A-D-E, X, n and Z are as defined in claim 1,
- **characterised in that** the secondary amine of formula II defined in claim 6, is reacted with :
. either a compound of formula V : 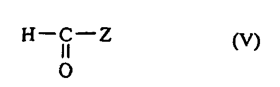 wherein Z is as defined above,
which is carried out in a reductive medium;
. or with a compound of formula VI : 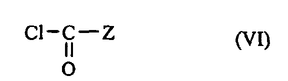
wherein Z is as defined above,
and the resulting compound of formula VII : 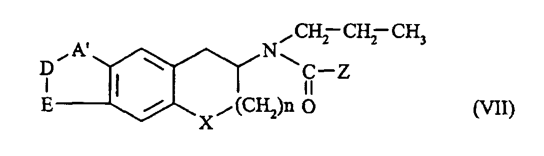 wherein A'-D-E, X, n and Z are as defined in claim 6, is reduced
- to obtain, whichever reaction route is used, a compound of formula VIII : 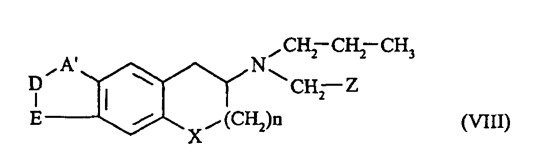 wherein A'-D-E, X, n and Z are as defined above ;
- which compound of formula VIII is then converted by oxidation by means :
. either of Jones reagent, or of 2,3-dichloro-5,6-dicyano-1,4-benzoquinone in acetic acid and water, when A'-D-E represents (CH₂)₃ or (CH₂)₄,
. or of hydrogen peroxide when -A'-D-E- represents -S-(CH₂)₂- or -S-CH=CH,
- to obtain the compounds of formula I"a : 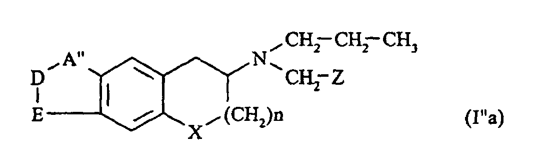 wherein A"-D-E, X, n and Z are as defined in claim 6,
- and, when A"-D-E represents 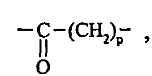 the corresponding compounds of formula I"a [that is to say the compounds corresponding more specifically to formula I'''a : 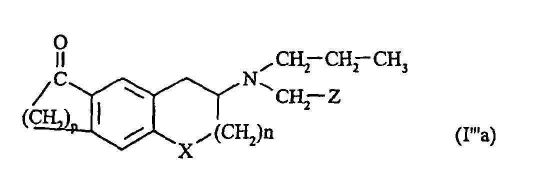
wherein p, X, n and Z are as defined in claim 1] are reduced
to obtain the compounds of formula I'b : 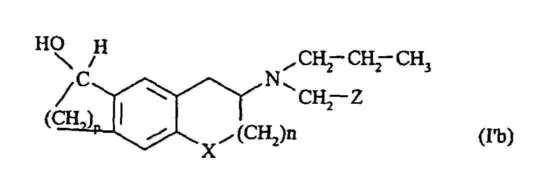 wherein p, X, n and Z are as defined above.

8. Process for the preparation of compounds of formula I" : 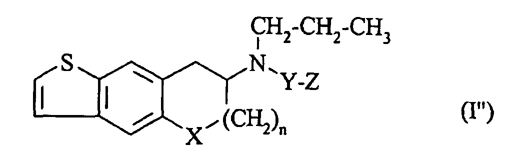 wherein X, n, Y and Z are as defined in claim 1,
**characterised in that** the compounds of formula IX : 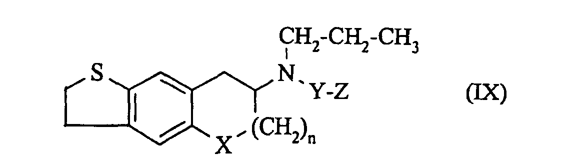 wherein X, n, Y et Z are as defined above,
are oxidised by means of hydrogen peroxide
to obtain the compounds of formula X : 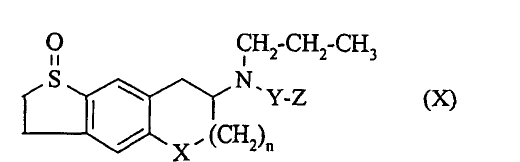 [wherein X, n, Y et Z are as defined above]
which are converted into compounds of formula I" by means of hydrochloric acid and ether.

9. A process for the preparation of compounds of formula I''' : 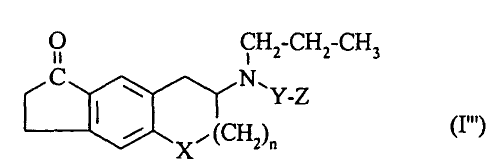 wherein X, n, Y and Z are as defined in claim 1,
**characterised in that** :
the compounds of formula XI : 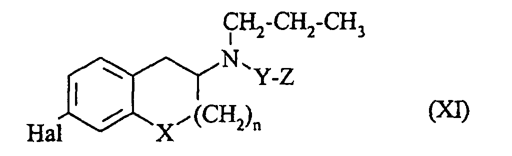
wherein X, n, Y and Z are as defined above, and
Hal represents a bromine or iodine atom,
are treated with methyl acrylate in dimethylformamide, in the presence of palladium acetate, tri-o-tolylphosphine and triethylamine,
to yield the compounds of formula XII : 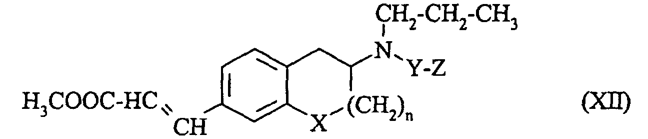 wherein X, n, Y and Z are as defined above,
which are reduced with hydrogen in the presence of platinum-on-carbon to yield the compounds of formula XIII : 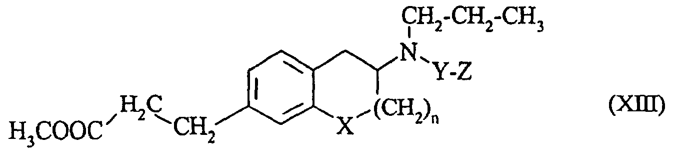 wherein X, n, Y and Z are as defined above,
which are treated with sodium hydroxide solution to yield the compounds of formula XIV : 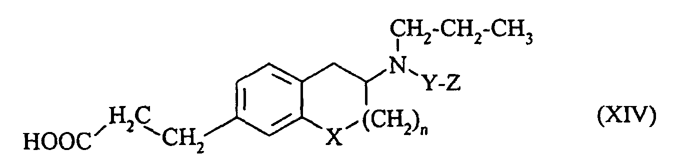 wherein X, n, Y and Z are as defined above,
which are cyclised by the action of phosphoric anhydride to obtain the compounds of formula I''' defined above.

10. Pharmaceutical compositions that can be used in the treatment of Parkinson's disease, memory disorders, disorders associated with drug abuse, depression and psychotic states, comprising as active ingredient a compound according to any one of claims 1 to 5 together with one or more appropriate pharmaceutical excipients.
